# EUROPÄISCHE PATENTANMELDUNG

(11) **EP 2 982 740 A1**
(43) Veröffentlichungstag der Anmeldung: **10.02.2016**
(21) Anmeldenummer: 15178587.0
(22) Anmeldetag: 28.07.2015
(51) Int. Cl.: C12M 1/107, C12M 1/00, C12P 5/02

(54) **VERFAHREN ZUR ERZEUGUNG VON METHAN**

(30) Priorität: 07.08.2014 DE 102014111287; 07.08.2014 DE 102014111298
(71) Anmelder: MicrobEnergy GmbH, 92421 Schwandorf (DE)
(72) Erfinder: Winter, Hans, 92269 Fensterbach/Wolfring (DE); Schmack, Doris, 93138 Lappersdorf (DE); Hackl, Florian, 93138 Lappersdorf (DE); Roth, Kerstin, 93158 Teublitz (DE); Reuter, Monika, 84561 Mehring (DE); Diller, Sabine, 92421 Schwandorf (DE); Beißinger, Martina, 93055 Regensburg (DE); Eichinger, Thomas, 92439 Bodenwöhr (DE)
(74) Vertreter: Glück Kritzenberger Patentanwälte PartGmbB

(57) **Zusammenfassung**

Beschrieben wird ein Verfahren zur Erzeugung von Methan umfassend die Schritte a) Bereitstellen einer Anlage zur Erzeugung von Biogas, wobei die Anlage zumindest eine Vorrichtung für die Zufuhr eines Substrats, zumindest eine Vorrichtung für die Abfuhr eines Gärrestes und zumindest einen Auslass für das in der Anlage zur Erzeugung von Biogas entstehende methan- und kohlendioxidhaltige Biogas aufweist, b) Bereitstellen eines Bioreaktors 14 zur Erzeugung eines methanangereicherten Gases, wobei der Bioreaktor 14 zumindest eine Vorrichtung für die Zufuhr des aus der Anlage zur Erzeugung von Biogas entnommenen Gärrestes, zumindest eine Vorrichtung für die Zufuhr des in der Anlage zur Erzeugung von Biogas entstehenden methan- und kohlendioxidhaltigen Biogases, zumindest eine Vorrichtung für die Zufuhr von Wasserstoff, zumindest einen Auslass für das in dem Bioreaktor entstehende methanangereicherte Gas und zumindest eine Temperiervorrichtung 18 aufweist, c) Herstellen von methan- und kohlendioxidhaltigem Biogas in der Anlage zur Erzeugung von Biogas, wobei das Herstellen des methan- und kohlendioxidhaltigen Biogases unter mesophilen Bedingungen erfolgt, d) Überführen zumindest eines Teils eines aus der Anlage zur Erzeugung von Biogas entnommenen Gärrestes in den Bioreaktor 14, e) Überführen des in Schritt c) hergestellten methan- und kohlendioxidhaltigen Biogases in den Bioreaktor 14, f) Zuführen von Wasserstoff in den Bioreaktor 14, g) Herstellen von methanangereichertem Gas in dem Bioreaktor (14), wobei der in Schritt d) überführte, aus der Anlage zur Erzeugung von Biogas entnommene Gärrest als Methanisierungsmedium dient, und wobei das Herstellen des methanangereicherten Gases in dem Bioreaktor (14) unter thermophilen Bedingungen erfolgt, h) Entnehmen des in dem Bioreaktor 14 gebildeten methanangereicherten Gases aus dem Bioreaktor.

## Beschreibung

### Technisches Gebiet

Die Erfindung betrifft ein Verfahren zur Erzeugung von Methan.

### Stand der Technik

Im Rahmen der Energiewende nehmen die erneuerbaren Energien einen immer größer werdenden Anteil an der erzeugten Energie ein. Da gerade erneuerbare Energieformen wie Strom aus Windkraft oder Photovoltaik sowohl zeitlich wie auch mengenmäßig nicht kontinuierlich und gleichmäßig zur Verfügung stehen und dabei auf Verbraucherseite ebenfalls auf eine zeitlich schwankende Nachfrage treffen, werden Speichermöglichkeiten für erneuerbare Energien zunehmend an Bedeutung gewinnen. Methan besitzt in diesem Zusammenhang eine hohe Bedeutung als chemischer Energieträger mit vielfältigen Einsatzmöglichkeiten in den Bereichen Wärmeerzeugung, Kraftstoffe oder Stromerzeugung.

Insbesondere in Energieumwandlungssystemen, die nach dem "Power-to-Gas"-Prinzip überschüssigen Strom in gasförmige chemische Energieträger wie Wasserstoff oder Methan umwandeln, erscheint Methan als geeigneter Energieträger, da es im Gegensatz zu Wasserstoff bei der vorhandenen Infrastruktur durch das Erdgasnetz sowie dem bereits bestehenden Netz an Erdgastankstellen eine gut speicherbare Energieform darstellt. Methan wird dabei aus den Ausgangsstoffen Kohlendioxid und Wasserstoff erzeugt. Der Wasserstoff wird in diesen Systemen in der Regel durch Elektrolyse von Wasser mit einem Elektrolyseur zur Verfügung gestellt, wobei Überschussstrom oder anderer günstiger Strom verwendet wird. Das notwendige Kohlendioxid für die Methanerzeugung kann aus verschiedenen Quellen wie Industrie- oder Verbrennungsabgasen kommen, bevorzugt wird jedoch klimafreundliches CO₂ aus erneuerbaren Quellen wie Biomasse verwendet, wie etwa aus Biogasanlagen oder auch Faulgas aus Kläranlagen.

Verfahren zur biologischen Methanisierung, bei denen das Biomethan durch methanogene Mikroorganismen gebildet wird, kommen im Vergleich zur chemisch katalytischen Methanisierung, beispielsweise nach dem Sabatier-Verfahren, ohne teure und empfindliche Katalysatoren aus und stellen geringere Anforderungen an Reaktionsbedingungen wie Temperatur und Druck sowie an die Reinheit der Ausgangsgase CO₂ und H₂.

Beispielsweise beschreibt die WO 2008/094282 A1 ein biologisches System zur Methanproduktion aus Wasserstoff und Kohlendioxid unter Verwendung einer Mikroorganismenkultur in einem Kulturmedium, welche mindestens eine Art von methanogenen Mikroorganismen enthält. Das Kohlendioxid kommt dabei aus einem industriellen Prozess, während der Wasserstoff u.a. durch Elektrolyse unter Einsatz von billigem Überschussstrom gewonnen werden kann.

Ferner wird gemäß WO 2012/094538 A1 eine biologische Methanproduktion aus CO₂ und H₂ in einem Bioreaktor offenbart, bei der ein spezieller Stamm des methanogenen Mikroorganismus *Methanothermobacter thermoautotrophicus* eingesetzt wird, der eine sehr langsame Wachstumsrate aufweist und nur wenig des angebotenen Kohlendioxids zum Biomasseaufbau verwendet, hingegen einen hohen Prozentsatz CO₂ in Methan umwandelt.

Ein System zur Speicherung von elektrischer Energie in Form von Methan ist aus der WO 2012/110257 A1 bekannt, wobei Strom aus erneuerbaren und nicht erneuerbaren Energien zur Wasserstoffproduktion verwendet wird. Der Wasserstoff wird zusammen mit Kohlendioxid in einen Reaktor eingeleitet, der Kulturen von methanogenen Mikroorganismen in einem Kulturmedium enthält, welche dann Methan produzieren.

Zur Erzielung einer wirtschaftlichen Betriebsweise sollte der Bioreaktor zur Erzeugung von Methan eine hohe Methanproduktivität bezogen auf das Reaktorvolumen liefern. Diese volumenspezifische Methanbildungsrate (angegeben in Normkubikmeter Methan pro Kubikmeter Reaktorvolumen pro Tag [Nm³/m³_{RV}d] bzw. Normliter Methan pro Liter Reaktorvolumen pro Tag [NI/I_{RV}d]) sollte dabei mindestens einen Wert von 10 Nm³/m³_{RV}d, bevorzugt einen Wert von mindestens 30 Nm³/m³_{RV}d, insbesondere von mindestens 50 Nm³/m³_{RV}d aufweisen. Die obenstehend im Stand der Technik beschriebenen technischen Verfahren und Systeme zur biologischen Methanisierung mit solchen Methanbildungsraten nutzen kontinuierlich betriebene Bioreaktoren, die mit Vorkulturen von speziellen, an die jeweiligen Reaktionsbedingungen angepassten methanogenen Mikroorganismen beimpft werden. Die entsprechenden Vorkulturen werden dabei unter den jeweils vorgesehenen Reaktionsbedingungen in geeigneten wässrigen Kulturmedien mit einer speziellen Zusammensetzung an Salzen, Nährstoffen und Spurenelementen gezüchtet, wobei diese Art der Vorkultivierung der methanogenen Mikroorganismen zur Beimpfung des Bioreaktors sowohl bezogen auf den laufenden Betrieb einer Anlage zur biologischen Methanisierung als auch auf die notwendige apparative Ausstattung der entsprechenden Anlage aufwändig und teuer ist. Beispielsweise laufen dabei Kosten für die Herstellung und den Austausch der Kulturmedien, für die Mediensterilisation, sowie für Anschaffung und Betrieb eines anaeroben Zuchtreaktors für methanogene Mikroorganismen auf.

Biogas, welches zum Beispiel in anaeroben Fermentern von Biogasanlagen oder in Faultürmen von Kläranlagen gebildet wird, enthält neben Methan und weiteren Gasen wie Sauerstoff oder Schwefelwasserstoff, die lediglich in kleiner Konzentration enthalten sind, einen Anteil an Kohlendioxid, der im Bereich von etwa 30 Vol.-% bis 55 Vol.-% liegt. Es bietet sich an, für eine biologische Methanisierung unter Zufuhr von externem Wasserstoff bevorzugt das im Biogas oder Faulgas enthaltene Kohlendioxid zu verwenden und so die Gesamtmethanausbeute des biologischen Prozesses deutlich zu steigern.

Beispielsweise offenbart die DE 10 2011 116 843 A1 ein entsprechendes Verfahren zur Erzeugung von Biogas, bei dem in einer ersten Gärstufe Biomasse unter Bildung eines Fermentationsrückstands und eines Rohbiogases vergoren wird. Wasserstoff und kohlendioxidhaltiges Gas wird in den Fermentationsrückstand einer zweiten oder späteren Gärstufe eingeleitet, um in einer biologischen Umwandlung den zugesetzten Wasserstoff mit dem Kohlendioxid aus dem Biogas in Methan umzusetzen.

Nachteilig an derartigen Verfahren oder Systemen ist, dass Biogasanlagen zur anaeroben Vergärung von Biomasse in der Regel eine volumenspezifische Methanbildungsrate von etwa 1 Nm³/m³_{RV}d aufweisen. Bei einem mittleren Rest-CO₂-Gehalt des Biogases von ca. 50 % wäre also im besten Fall bei einer biologischen Methanisierung unter Zufuhr von extern gebildetem Wasserstoff eine zusätzliche volumenspezifische Methanbildungsrate von etwa 1 Nm³/m³_{RV}d, also insgesamt 2 Nm³/m³_{RV}d zu erreichen. Diese kann nur dann erreicht werden, wenn in den Fermentern der Biogasanlage das komplette Rest-CO₂ mit zugeführtem Wasserstoff zu Methan umgewandelt werden würde. Methanbildungsraten von 10 Nm³/m³_{RV}d oder mehr sind somit in den Fermentern einer Biogasanlage nicht erreichbar. Daneben hat sich gezeigt, dass eine effiziente Wasserstoffeinbringung in anaerobe Fermenter von konventionellen Biogasanlagen kaum durchführbar ist.

Für Kläranlagen würde die entsprechende Bilanz noch schlechter ausfallen, da dort in der Regel Methanbildungsraten von deutlich unter 1 Nm³/m³_{RV}d auftreten und der Rest-CO₂-Gehalt des Faulgases gewöhnlich unter 50 %, eher im Bereich von 30 % liegt.

In Weiterentwicklung dessen sind Verfahren zur Erzeugung von Biomethan bekannt, die in Kombination mit vorhandenen Biogasanlagen separate Bioreaktoren zur biologischen Methanisierung betreiben, um unter Verwendung des kohlendioxidhaltigen Rohbiogases ein Biogas mit einem erhöhten Methangehalt zu produzieren, was in Fachkreisen auch als so genanntes "Biogas Upgrading" bezeichnet wird.

In der WO 2013/060331 A1 wird beispielsweise ein solcher Prozess zum Biogas Upgrading beschrieben, bei dem mit Zusatz von externem Wasserstoff und unter Verwendung eines sauren Biomasseabfallstoffs als Kosubstrat aus Biogas ein verbessertes Biogas mit höherem Methangehalt hergestellt wird. Dabei wird beispielsweise Wasserstoff und Rohbiogas in einen zweiten Reaktor eingeleitet, wobei für die biologische Methanisierung in den zweiten Reaktor ferner Nährstoffe und ein Inokulum enthaltend hydrogenotrophe methanogene Mikroorganismen eingebracht werden.

Die DE 10 2011 054 298 A1 offenbart eine Methanerzeugungseinheit in Form eines Methanisierungsreaktors, in dem Oberflächen angeordnet sind, an denen immobilisierte methanogene Mikroorganismen Methan erzeugen, dadurch dass dem flüssigen Reaktorinhalt ausschließlich gasförmige Substrate in Form von Wasserstoff bzw. Kohlendioxid und Kohlenmonoxid zugeführt werden. Als transportabel ausgebildete Einheit ist dieser Methanisierungsreaktor auch in eine Biogasanlage integrierbar, insbesondere in Verbindung mit einer Ammoniakstripeinheit sowie -spalteinheit.

Die DE 10 2013 001 689 A1 beschreibt ein Verfahren zur Herstellung von Biomethan, wobei der Bio-Methanisierungs-Reaktor mit einer Bakteriensuspension aus einem Fermenter einer Biogasanlage oder aus dem Faulturm einer Kläranlage beschickt wird und als gasförmiges Substrat Biogas oder Faulgas und Wasserstoff hinzugegeben werden. Die Bakteriensuspension wird bevorzugt immobilisiert und die Gasphase im Gegenstrom zur Suspensionsphase geführt.

Die GB 2476090 A beschreibt ein Verfahren, bei dem Wasserstoff und Kohlendioxid kombiniert werden, um Methan und andere Kohlenwasserstoffe als Kraftstoffe zu produzieren. Neben externem Wasserstoff wird in den anaeroben Fermenter auch eine Lösung mit gelöstem Kohlendioxid oder Karbonaten zugegeben.

Derzeit gibt es einige Demonstrations- oder Pilotprojekte, die zeigen, dass biologische Methanisierung im Rahmen des "Power to Gas"-Ansatzes prinzipiell machbar ist. Kurzfristig steht jedoch keine großtechnisch verfügbare wirtschaftlich nutzbare Technologie zur Verfügung, mit der sich die biologische Methanisierung am Markt etablieren könnte. Deshalb ist es trotz dieser Lösungsansätze notwendig, einfache und kosteneffiziente Systeme zur Verfügung zu stellen, die sich gut in vorhandene Strukturen und Abläufe integrieren lassen, wobei mit möglichst geringem Aufwand und geringen Kosten eine hohe Methanausbeute erreicht werden sollte.

### Darstellung der Erfindung

Der Erfindung, wie sie in den Ansprüchen gekennzeichnet ist, liegt die Aufgabe zu Grunde, ein einfaches, wirtschaftliches und kostengünstiges Verfahren bereitzustellen, das geeignet ist, unter Verwendung eines CO₂-haltigen Gases sowie unter Verwendung von gasförmigem Wasserstoff in effizienter Weise Biomethan zu erzeugen.

Diese Aufgabe wird erfindungsgemäß durch das Verfahren zur Erzeugung von Methan gemäß Anspruch 1 gelöst. Weitere vorteilhafte Details, Aspekte und Ausgestaltungen der vorliegenden Erfindung ergeben sich aus den abhängigen Ansprüchen, der Beschreibung und den Beispielen.

Die vorliegende Erfindung stellt ein Verfahren zur Erzeugung von Methan zur Verfügung. Das Verfahren umfasst die Schritte a) Bereitstellen einer Anlage zur Erzeugung von Biogas, wobei die Anlage zumindest eine Vorrichtung für die Zufuhr eines Substrats, zumindest eine Vorrichtung für die Abfuhr eines Gärrestes und zumindest einen Auslass für das in der Anlage zur Erzeugung von Biogas entstehende methan- und kohlendioxidhaltige Biogas aufweist, b) Bereitstellen eines Bioreaktors zur Erzeugung eines methanangereicherten Gases, wobei der Bioreaktor zumindest eine Vorrichtung für die Zufuhr des aus der Anlage zur Erzeugung von Biogas entnommenen Gärrestes, zumindest eine Vorrichtung für die Zufuhr des in der Anlage zur Erzeugung von Biogas entstehenden methan- und kohlendioxidhaltigen Biogases, zumindest eine Vorrichtung für die Zufuhr von Wasserstoff, zumindest einen Auslass für das in dem Bioreaktor entstehende methanangereicherte Gas und zumindest eine Temperiervorrichtung aufweist, c) Herstellen von methan- und kohlendioxidhaltigem Biogas in der Anlage zur Erzeugung von Biogas, wobei das Herstellen des methan- und kohlendioxidhaltigen Biogases unter mesophilen Bedingungen erfolgt, d) Überführen zumindest eines Teils eines aus der Anlage zur Erzeugung von Biogas entnommenen Gärrestes in den Bioreaktor, e) Überführen des in Schritt c) hergestellten methan- und kohlendioxidhaltigen Biogases in den Bioreaktor, f) Zufuhr von Wasserstoff in den Bioreaktor, g) Herstellen von methanangereichertem Gas in dem Bioreaktor, wobei der in Schritt d) überführte, aus der Anlage zur Erzeugung von Biogas entnommene Gärrest als Methanisierungsmedium dient und wobei das Herstellen des methanangereicherten Gases in dem Bioreaktor unter thermophilen Bedingungen erfolgt und h) Entnehmen des in dem Bioreaktor gebildeten methanangereicherten Gases aus dem Bioreaktor.

Das erfindungsgemäße Verfahren wird mit Hilfe einer Anlage zur Erzeugung von Biogas durchgeführt, die durch einen Bioreaktor zur Erzeugung eines methanangereicherten Gases mittels biologischer Methanisierung ergänzt ist. Dieser Bioreaktor wird mit dem in der Anlage zur Erzeugung von Biogas erzeugten methan- und kohlendioxidhaltigen Biogas sowie mit extern erzeugtem Wasserstoff gespeist. Als Substrat in dem Bioreaktor wird ausschließlich Gärrest aus der Anlage zur Erzeugung von Biogas verwendet, so dass im laufenden bzw. kontinuierlichen Betrieb während der Bildung des methanangereicherten Gases in dem Bioreaktor außer dem Gärrest kein weiteres Substrat im Sinne von zusätzlicher Biomasse oder Nährmedien bzw. -lösungen anwesend ist. Ebenso werden dem Bioreaktor im laufenden bzw. kontinuierlichen Betrieb neben den im Gärrest naturgemäß enthaltenen Mikroorganismen keine weiteren oder zusätzlichen Mikroorganismen zugeführt. Der Bioreaktor zur biologischen Methanisierung weist vorteilhafterweise ein geeignetes System zur Gaseinbringung in das flüssige Reaktormedium für das CO₂-haltige Biogas sowie für den Wasserstoff auf. Das gebildete methanangereicherte Gas wird über eine Produktgasleitung aus dem Bioreaktor abgeführt.

Völlig überraschend hat sich gezeigt, dass sich der Gärrest aus einer mesophil betriebenen Anlage zur Erzeugung von Biogas in hervorragender Weise als Methanisierungsmedium, nämlich zugleich als Inokulum und als Substrat für einen unter thermophilen Bedingungen betriebenen Bioreaktor zur biologischen Methanisierung eignet. Erfindungsgemäß erfolgt daher das Herstellen des methan-und kohlendioxidhaltigen Biogases unter mesophilen Bedingungen und das Herstellen des methanangereicherten Gases in dem Bioreaktor unter thermophilen Bedingungen.

Wie bereits einleitend erwähnt, wird bei einer Biomethanisierung eine volumenspezifische Methanbildungsrate angestrebt, die ein Vielfaches der Methanbildungsrate beträgt, wie sie in anaeroben Fermentern von Biogasanlagen oder in Faultürmen von Kläranlagen gegeben ist. Dies ist notwendig, um die Bioreaktoren für die biologische Methanisierung kompakt zu gestalten, um so Investitionskosten zu sparen, insbesondere, da der günstige Strom für die Wasserstoffelektrolyse häufig nicht kontinuierlich zur Verfügung steht und bei einer geringen Betriebsstundenzahl einer Anlage die Investitionskosten überproportional zu den Gesamtkosten beitragen. Um hohe Methanbildungsraten von größer als 10 Nm³/m³_{RV}d zu erreichen, sind in den Bioreaktoren zur Methanisierung andere Reaktionsbedingungen notwendig als sie gewöhnlich in anaeroben Fermentern von Biogasanlagen oder Faultürmen von Kläranlagen herrschen. Gemäß der vorliegenden Erfindung wird die biologische Methanisierung unter thermophilen Bedingungen, also bei Temperaturen höher als 45 °C betrieben.

Um hohe Kosten für die Herstellung von synthetischen Kulturmedien für die hydrogenotrophen methanogenen Mikroorganismen zu vermeiden, wird als Kulturmedium Material verwendet, das in Anlagen zur Erzeugung von Biogas ohnehin vorhanden ist und letztendlich als Gärrest anfällt. Erfindungsgemäß dient dieser anfallende Gärrest als Methanisierungsmedium und kann zwar für die biologische Methanisierung in dem separaten Bioreaktor adaptiert werden, jedoch sind dies einfache und kostengünstige Maßnahmen, wie die Verdünnung des Gärsubstrats oder die Zugabe von Puffersalzen.

Im Gegensatz zu dem vorliegenden erfindungsgemäßen Verfahren müssen bei den aus dem Stand der Technik bekannten Verfahren oder Systemen zur Erzeugung von Methan, die mit synthetischen Kulturmedien arbeiten, hydrogenotrophe methanogene Mikroorganismen in entsprechenden Vorkulturen angereichert und dann von extern in den Bioreaktor zur biologischen Methanisierung zugegeben werden. Der erfindungsgemäß als Methanisierungsmedium verwendete Gärrest aus einer Anlage zur Erzeugung von Biogas ist jedoch besonders vorteilhaft selbst in der Lage, Methan zu bilden, ohne externe Zugabe von Kulturen an methanogenen Mikroorganismen.

In einer Anlage zur Erzeugung von Biogas erfolgt die Biogasbildung innerhalb einer mikrobiellen Gesellschaft mit vielen nicht Methan bildenden Mikroorganismen, die beispielsweise längerkettige Makromoleküle hydrolysieren oder Acetat und andere Fettsäuren bilden. In der Praxis werden die meisten Anlagen zur Erzeugung von Biogas (ca. 85 %) in einem mesophilen Temperaturbereich zwischen 32 °C und 42 °C betrieben (siehe beispielsweise Handreichung Biogasgewinnung und -nutzung, Fachagentur für nachwachsende Rohstoffe, 2006; S. 27, 1. Absatz; S. 37, 1. Absatz). Insofern ist es aus ökonomischen und verfahrenstechnischen Gründen wünschenswert, für die biologische Methanisierung in einem separaten Bioreaktor den Gärrest aus diesen mesophil betriebenen Anlagen nutzen zu können.

Soll nun ein entsprechender Bioreaktor zur biologischen Methanisierung, in dem der CO₂-Anteil aus dem Biogas als gasförmiges Substrat zur Methanisierung genutzt wird, aus den oben dargelegten verfahrenstechnischen Erwägungen thermophil betrieben werden, so scheint nach allgemeinem Fachwissen und dem bekannten Stand der Technik eine Kopplung der thermophilen Biomethanisierung mit der oberhalb erwähnten mesophilen Biogaserzeugung inkompatibel zu sein, da die in den vorliegenden Gärsubstraten vorherrschenden Populationen von Mikroorganismen nicht an thermophile Bedingungen angepasst sind.

Ein mikrobiologisch ausgebildeter oder in der Biogas- bzw. Abwasserbranche tätiger Fachmann würde für den Betrieb eines Methanisierungsreaktors unter thermophilen Bedingungen ein Inokulum aus einer ebenso thermophil betriebenen Anlage benutzen oder spezielle Anreicherungskulturen oder Reinkulturen von thermophilen Methanbildnern zusetzen. Beispielsweise wird nach empfohlener und fachkundlicher Vorgehensweise beim Anfahren einer Biogasanlage als Inokulum zum Animpfen Material aus einer anderen Biogasanlage benutzt, die unter möglichst ähnlichen Bedingungen hinsichtlich der Substratzusammensetzung und der Betriebsweise, insbesondere des Temperaturniveaus, betrieben wird (siehe "Hinweise zum Anfahren einer Biogasanlage", BiogasForum Bayern, Nr. III - 2/2009, insbesondere S. 2 und S. 4).

Bei den eingangs genannten, aus dem Stand der Technik bekannten Verfahren zur Erzeugung von Methan, bei denen Gärsubstrate in separaten Bioreaktoren zur biologischen Methanisierung verwendet werden, wird die Betriebstemperatur des Methanisierungsreaktors an die in den jeweiligen Verfahren verwendeten, teilweise immobilisierten Bakterien angepasst (vergleiche dazu z.B. DE 10 2011 054 298 A1 auf Seite 6, linke Spalte, untere Hälfte erster Absatz oder GB 2476090 A auf Seite 1, Absatz 8). Dies bedeutet, dass für solche unter mesophilen Bedingungen betriebenen Methanisierungsreaktoren Gärsubstrate aus mesophil betriebenen Anlagen zur Erzeugung von Biogas als Inokulum bzw. Anreicherungskulturen von mesophilen Methanogenen verwendet werden und entsprechend analog Gärsubstrate aus thermophil betriebenen Anlagen zur Erzeugung von Biogas in Kombination mit thermophil betriebenen Methanisierungsreaktoren verwendet werden (siehe dazu auch WO 2013/060331 auf Seite 34, Zeilen 4 bis 6, Zeilen 9 bis 10, Zeilen 16 bis 18 und Zeilen 24 bis 26), was im Einklang mit dem fachmännischen Wissen steht.

Nach allgemeinem Fachwissen trifft die beschriebene Temperaturproblematik insbesondere für die Biogas- oder Biomethanproduktion unter thermophilen Bedingungen zu, da ein thermophiler Prozess in diesem Zusammenhang im Allgemeinen weniger stabil verläuft und insgesamt störanfälliger ist. Da die mikrobiellen Gemeinschaften bei mesophiler und thermophiler Biogasproduktion sehr unterschiedlich sind, ist es nicht möglich, vom Verständnis der Prozesse unter mesophilen Bedingungen einfach auf solche unter thermophilen Bedingungen zu extrapolieren.

Besonders vorteilhaft wird mit dem erfindungsgemäßen Verfahren überraschend und unerwartet durch Hintereinanderschaltung einer unter mesophilen Bedingungen stattfindenden Herstellung von methan- und kohlendioxidhaltigem Biogas und einer unter thermophilen Bedingungen stattfindenden Herstellung eines methanangereicherten Gases eine gesteigerte und hocheffiziente volumenspezifische Methanbildungsrate erreicht und zwar insbesondere ohne die Verwendung zusätzlichen Substrates und/oder zusätzlicher Mikroorganismen. Eine für einen Fachmann erwartete Temperaturproblematik, nämlich dass die Herstellung von methanangereichertem Gas unter thermophilen Bedingungen nur sehr schlecht bzw. sehr langsam und mit geringer Effizienz oder gar nicht funktioniert, wenn als Inokulum ein Gärrest aus einer unter mesophilen Bedingungen betriebenen Anlage zur Erzeugung von Biogas verwendet wird, ist bei dem erfindungsgemäßen Verfahren überraschenderweise nicht zu beobachten. Entgegen den Erwartungen des Fachmannes kann mit dem erfindungsgemäßen Verfahren eine rasch anlaufende Methanisierung mit einer hohen Methanausbeute und Methanbildungsrate erzielt werden, obwohl dem Grundsatz widersprochen wird, dass in einem thermophil betriebenen Bioreaktor zur Erzeugung eines methanangereicherten Gases nur ein ebenso unter thermophilen Bedingungen gewonnenes Inokulum verwendet werden kann.

### Definitionen:

*Biogas:* Unter Biogas ist im Folgenden ein Gas zu verstehen, das in einer anaeroben Fermentation in einer Anlage zur Erzeugung von Biogas unter Einwirkung von verschiedenen Mikroorganismen gebildet wird. Es enthält als Hauptbestandteile Methan und Kohlendioxid. Daneben enthält es Wasserdampf und gegebenenfalls kleine Anteile an Wasserstoff, Stickstoff, Sauerstoff, Schwefelwasserstoff und Ammoniak. Biogas wird im Rahmen der vorliegenden Anmeldung auch als "methan- und kohlendioxidhaltiges Biogas" bezeichnet. Eine Anlage zur Erzeugung von Biogas kann beispielsweise eine Biogasanlage sein, in der das Biogas aus Biomasse erzeugt wird. Ferner kann die Anlage zur Erzeugung von Biogas auch eine Kläranlage (Definition siehe unten) sein, welche vorzugsweise einen Faulturm aufweist. Das in einer Kläranlage erzeugte Biogas wird vorliegend auch als Faulgas oder Klärgas (Definition siehe unten) bezeichnet.

*Kläranlage:* Unter Kläranlage ist eine Abwasseraufbereitungsanlage zu verstehen. Kommunale Kläranlagen bereiten hauptsächlich organisch belastetes Abwasser aus der Kanalisation auf, nehmen teilweise jedoch auch leicht abbaubaren organischen Abfall an. Industrieelle Kläranlagen arbeiten spezielle, in der jeweiligen Industrie anfallende organisch belastete Abwässer auf.

*Rohbiogas:* Mit Rohbiogas wird das Biogas bezeichnet, welches direkt aus einer Anlage zur Erzeugung von Biogas stammt und nicht durch eine Biogasaufbereitungsanlage weiter aufbereitet wurde.

*Faulgas:* Unter Faulgas oder Klärgas ist im Folgenden ein Biogas zu verstehen, das in einer anaeroben Fermentation bei der Schlammstabilisierung in einem Faulturm aus Rohschlamm unter Einwirkung von verschiedenen Mikroorganismen gebildet wird. Es enthält als Hauptbestandteile Methan und Kohlendioxid. Daneben enthält es Wasserdampf und gegebenenfalls kleine Anteile an Wasserstoff, Stickstoff, Sauerstoff, Schwefelwasserstoff, weiteren Spurenkomponenten wie Siloxane oder aromatische Kohlenwasserstoffe und Ammoniak. Faulgas wird im Rahmen der vorliegenden Anmeldung auch als "methan- und kohlendioxidhaltiges Biogas" oder "methan- und kohlendioxidhaltiges Faulgas" bezeichnet.

*Rohfaulgas:* Mit Rohfaulgas wird das Faulgas bezeichnet, welches direkt aus einem oder mehreren anaeroben Faultürmen der Kläranlage stammt und nicht durch eine Faulgasaufbereitungsanlage weiter aufbereitet wurde.

*Dünnschlamm:* Als Dünnschlamm wird der flüssige Gärrestbestandteil aus Biogasanlagen bezeichnet, der nach einer Fest-Flüssig-Separation des Gärrestes, insbesondere durch mechanische Separation, erhalten wird. Dünnschlamm kann entweder als Dünger weiterverwendet und so wieder in den Kohlenstoffkreislauf eingebracht werden oder er wird innerhalb der Biogasanlage rezirkuliert und gelangt so erneut in die anaeroben Fermenter zur Biogasgewinnung.

*Klärschlamm:* Klärschlamm entsteht bei der Abwasserreinigung und ist eine Mischung aus Wasser und Feststoffen. Man unterscheidet zwischen Rohschlamm und Faulschlamm. Rohschlamm fällt auf Kläranlagen als Primärschlamm in der mechanischen Reinigungsstufe oder als Überschussschlamm in der biologischen Stufe an. Eine Stabilisierung des Klärschlamms wird erreicht, indem die biologisch abbaubare Substanz des Klärschlamms durch mikrobielle Stoffwechselprozesse soweit reduziert wird, dass Geruchsemissionen und andere Beeinträchtigungen der Umwelt weitgehend ausgeschlossen sind. Diese Behandlung erfolgt in größeren Kläranlagen in Faultürmen.

*Rohschlamm:* Als Rohschlamm bezeichnet man unbehandelten Klärschlamm, der noch nicht stabilisiert wurde. Rohschlamm fällt auf Kläranlagen als Primärschlamm oder Überschussschlamm an.

*Primärschlamm:* Primärschlamm fällt als Klärschlamm aus der ersten mechanischen Reinigungsstufe an.

*Überschussschlamm:* Überschussschlamm oder auch Sekundärschlamm entsteht bei der biologischen Reinigung des Abwassers in einer aeroben Stufe und ist reich an Mikroorganismen. Gegebenenfalls fallen bei weiteren Reinigungsstufen entsprechend Tertiär- oder Quartärschlämme an.

*Faulschlamm:* Nach dem Ausfaulen in so genannten Faultürmen ist der Rohschlamm stabilisiert und wird als Faulschlamm bezeichnet, der nur noch eine geringe Geruchsbelästigung verursacht. Er enthält nur 1 bis 5 Prozent Trockenmasse und muss deshalb noch entwässert werden. Findet eine Schlammseparation durch eine mechanische Entwässerung statt, wird der Faulschlamm in feste (hoher Trockensubstanzgehalt) und flüssige Anteile (niedriger Trockensubstanzgehalt; Schlammwasser, Trübwasser) aufgetrennt. Ohne Schlammseparation verbleibt ein Faulschlamm mit einem Trockensubstanzgehalt (TS-Gehalt), der zwischen dem eines festen und dem eines flüssigen Faulschlammbestandteils liegt. Diesem Faulschlamm muss durch biologische Entwässerung oder Klärschlammtrocknung Wasser entzogen werden.

*Biogasbildung:* Unter Biogasbildung wird die Produktion eines Gases mit Methan und Kohlendioxid als wesentlichen Bestandteilen verstanden, die in einer anaerob betriebenen Anlage einhergehend mit einem Abbau von Biomasse erfolgt und bei der eine Vielzahl von verschiedenen Mikroorganismen sowie unterschiedliche Stoffwechselwege (beispielsweise Hydrolyse, Acidogenese, Acetogenese, Methanbildung) beteiligt sind. Biogas kann in einer einstufigen Biogasanlage in einem anaeroben Fermenter gebildet werden, in mehrstufigen Biogasanlagen jedoch auch in unterschiedlichen Zusammensetzungen in speziellen anaeroben Fermentern wie Hydrolysefermenter, Methanisierungsfermenter oder Nachgärer. Ferner kann Biogas in einem anaerob betriebenen Faulturm einer Kläranlage gebildet werden. Das dabei entstehende Biogas wird auch als Faulgas bezeichnet, die Biogasbildung kann dabei auch als Faulgasbildung verstanden werden. *Biomethan:* Unter Biomethan ist im Folgenden ein Methan zu verstehen, das durch die Einwirkung von hydrogenotrophen methanogenen Mikroorganismen in einem anaeroben Bioreaktor unter Zufuhr von wasserstoffhaltigem und kohlendioxidhaltigem Gas gebildet wird. Der Begriff Biomethan ist als Abgrenzung zu synthetischem Methan zu verstehen, das bei der chemisch katalytischen Methanisierung gebildet wird. Das gemäß der vorliegenden Erfindung produzierte Biomethan kann neben CH₄ auch Anteile aus den in dem Bioreaktor zur Methanisierung eingebrachten Eduktgasen, beispielsweise von nicht umgesetztem Wasserstoff und Kohlendioxid enthalten, so dass es nicht zu 100 % aus Methan bestehen muss. Methan ist jedoch der Hauptbestandteil von Biomethan. Im Rahmen der vorliegenden Anmeldung wird Biomethan auch als "methanangereichertes Gas" bezeichnet und kann ebenso als methanangereichertes Biogas verstanden werden.

*Bioerdgas:* Unter Bioerdgas wird ein Methangas verstanden, das nach den jeweils gültigen Richtlinien (in Deutschland beispielsweise DVGW-Richtlinien G260, G262) ins Erdgasnetz eingespeist werden kann und direkt aus Biomasse (einspeisefähiges Biogas) oder in einer biologischen Methanisierung unter Verwendung von CO₂ biogenen Ursprungs erzeugt wurde (einspeisefähiges Biomethan).

*Biomasse:* Unter Biomasse im Sinne der Erfindung werden alle Arten von vergärbaren nachwachsenden Rohstoffen wie Mais, Getreide, Gras, Silphie, Zuckerrüben, aber auch tierische Exkremente wie Rinder- oder Schweinegülle, Pferdemist oder Hühnertrockenkot sowie Bioabfälle oder organische Abfälle aus der Lebensmittelproduktion oder -verarbeitung sowie beliebige Mischungen aus diesen Gärsubstraten verstanden.

*Gärrest:* Als Gärrest oder Gärrückstand wird der Rückstand an Gärsubstrat bezeichnet, der bei der Vergärung von Biomasse in einer Anlage zur Erzeugung von Biogas zurückbleibt. Unter Gärrest wird im vorliegenden Sinne beispielsweise der Rückstand nach der letzten temperierten Fermenterstufe (z.B. Nachgärer) in einer Biogasanlage verstanden. Ebenso wird unter Gärrest im Sinne der vorliegenden Erfindung Faulschlamm aus Faultürmen von Kläranlagen verstanden. Findet eine Gärresteseparation statt, wird der Gärrest in feste (hoher Trockensubstanzgehalt) und flüssige Anteile (niedriger Trockensubstanzgehalt) aufgetrennt. Ohne Gärrestseparation verbleibt ein Gärrest mit einem Trockensubstanzgehalt (TS-Gehalt), der zwischen dem eines festen und dem eines flüssigen Gärrestbestandteils liegt. Der Gärrest wird in einem Endlager, Gärrestbehälter oder Substratbehälter gelagert. Da auch im Gärrest noch ein Biogasrestpotential vorhanden ist, sind zumindest in neueren Anlagen zur Erzeugung von Biogas, insbesondere Biogasanlagen die Gärrestbehälter gasdicht ausgeführt, so dass entstehendes Biogas ebenfalls abgeführt und der Rohbiogasleitung zugeführt wird.

*Methanisierung:* Unter Methanisierung ist eine Methanbildung ausgehend von den gasförmigen Stoffen Wasserstoff und Kohlendioxid als Eduktgase zu verstehen. Biologische Methanisierung beschreibt die Bildung von Biomethan mit Hilfe von hydrogenotrophen methanogenen Mikroorganismen in einem wässrigen Medium. Sie erfolgt nach der chemischen Gleichung: 4 H₂ + CO₂ → CH₄ + 2 H₂O.

*Methanisierungsmedium:* Bezeichnet den Reaktorinhalt des Bioreaktors, der geeignet ist, eine biologische Methanisierung durchzuführen. Das Methanisierungsmedium beinhaltet ein komplexes Medium, das sich aus Gärrest aus der Anlage zur Erzeugung von Biogas sowie optional aus wässrigen Lösungen zur Verdünnung desselben zusammensetzt. Es beinhaltet alle Nährstoffe und Spurenelemente, die für das Wachstum entsprechender hydrogenotropher methanogener Mikroorganismen nötig sind sowie die hydrogenotrophen methanogenen Mikroorganismen selbst. Im Rahmen der vorliegenden Anmeldung ist unter dem Methanisierungsmedium folglich der aus der Anlage zur Erzeugung von Biogas entnommene Gärrest zu verstehen, welcher sowohl das für die Methanbildung benötigte Substrat als auch die dazu notwendigen hydrogenotrophen methanogenen Mikroorganismen enthält und welcher im Wesentlichen unverändert dem Bioreaktor zugeführt wird. Eine optionale Adaptation des Methanisierungsmediums beschränkt sich auf eine Verdünnung des Gärrestes mit Wasser oder mit wässrigen Lösungen oder auf die Zugabe von Salzen und/oder Spurenelementen zu dem Gärrest.

*Mesophil:* Unter mesophilen Bedingungen wird im Sinne der vorliegenden Erfindung verstanden, dass eine Temperatur in einem Temperaturbereich zwischen 20°C bis 42°C, bevorzugt zwischen 30°C und 37°C eingestellt und gehalten wird. Mesophile Bedingungen werden vorliegend beispielsweise in der Anlage zur Erzeugung von Biogas bei der Herstellung von Biogas eingestellt. Als mesophile Organismen werden im Sinne der vorliegenden Erfindung Organismen verstanden, die in etwa im Temperaturbereich warmblütiger Tiere leben und ihr Wachstumsoptimum in einem Bereich zwischen 20°C bis 42°C haben (vgl. Brock Mikrobiologie, 11. Auflage, 2009; S. 1171 und Allgemeine Mikrobiologie, Hans G. Schlegel, 6. Überarbeitete Auflage, 1985, Seite 178).

*Thermophil:* Unter thermophilen Bedingungen wird im Sinne der vorliegenden Erfindung verstanden, dass eine Temperatur in einem Temperaturbereich zwischen 45°C und 100°C, bevorzugt zwischen 50°C und 80°C und insbesondere bevorzugt zwischen 60°C und 75°C eingestellt und gehalten wird. Thermophile Bedingungen werden vorliegend beispielsweise in dem Bioreaktor zur Erzeugung von methanangereichertem Gas bei der biologischen Methanisierung eingestellt. Als thermophile Organismen werden im Sinne der vorliegenden Erfindung Organismen bezeichnet, die ihr Wachstumsoptimum im Temperaturbereich zwischen 45°C und 80°C haben (vgl. Brock Mikrobiologie, 11. Auflage, 2009; S. 172, S. 1178).

Vorzugsweise wird mit dem Verfahren eine biologische Methanisierung im Kontext einer zumindest einen Fermenter aufweisenden Biogasanlage durchgeführt. Ein damit verbundener Vorteil ist, dass der eigentlich als Abfallprodukt anfallende CO₂₋Anteil des in der Biogasanlage aus vergorener Biomasse entstandenen Biogases durch die biologische Methanisierung energetisch zu Biomethan aufgewertet wird, so dass der Gesamtertrag an Methan in der Biogasanlage steigt. Zudem hat sich gezeigt, dass der Bioreaktor, in dem das methanangereicherte Gas gebildet wird, ausschließlich mit dem in der Biogasanlage anfallenden Gärrest als Substrat betrieben werden kann. Dieses eigentlich als "Abfall" der Biogasanlage anfallende biologische Material wird damit unmittelbar einer weiteren Verwertung zugeführt.

Alternativ und ebenso bevorzugt wird mit dem Verfahren eine biologische Methanisierung im Kontext einer zumindest einen Faulturm aufweisenden Kläranlage durchgeführt. Ein damit verbundener Vorteil ist, dass der eigentlich als Abfallprodukt anfallende CO₂-Anteil des in einer Kläranlage aus organisch belastetem Abwasser entstandenen CO₂-reichen Faulgases durch die biologische Methanisierung energetisch zu Biomethan aufgewertet wird. Zudem hat sich gezeigt, dass der Bioreaktor, in dem das methanangereicherte Gas gebildet wird, ausschließlich mit dem in der Kläranlage anfallenden Rohschlamm und/oder Faulschlamm als Substrat betrieben werden kann. Dieses eigentlich als "Abfall" der Kläranlage anfallende biologische Material wird damit unmittelbar einer weiteren Verwertung zugeführt.

Es ist ebenso denkbar, den Bioreaktor zur Bildung des methanangereicherten Gases auf einer Biogasanlage mit Klärschlamm aus einer Kläranlage als Substrat zu betreiben und auch umgekehrt, den Bioreaktor zur Bildung des methanangereicherten Gases auf einer Kläranlage mit Gärrest aus einer Biogasanlage zu betreiben. Dies ist jedoch aufwändiger in der Handhabung der Gärreste, so dass dies zwar eine mögliche aber keine besonders bevorzugte Ausführungsform darstellt.

Das methan- und kohlendioxidhaltige Biogas wird unter mesophilen Bedingungen hergestellt, während im Bioreaktor zur Herstellung des methanangereicherten Gases durch biologische Methanisierung thermophile Bedingungen herrschen. Bevorzugt wird dabei in einem thermophilen Bereich bei Temperaturen oberhalb von 45 °C methanisiert, insbesondere bei Temperaturen von 50 °C bis 100 °C, besonders bevorzugt bei Temperaturen von 60 °C bis 75 °C. Die vorgesehene Temperiervorrichtung ist geeignet, die entsprechende Temperatur für die biologische Methanisierung nach Vorgabe einzustellen und wird vorzugsweise derart betrieben, dass der in dem Bioreaktor vorliegende, aus der Anlage zur Erzeugung von Biogas entnommene Gärrest auf einer Temperatur T ≥ 45 °C, bevorzugt 50 °C ≤ T ≤ 100 °C, besonders bevorzugt 60 °C ≤ T ≤ 75 °C gehalten wird. Die Temperiervorrichtung ist dazu ausgelegt und eingerichtet den Reaktoreinhalt des Bioreaktors sowohl zu erwärmen als auch unter besonderen Umständen zu kühlen. Ebenso kann die Temperiervorrichtung so ausgelegt sein, dass auch der Inhalt der Anlage zur Erzeugung von Biogas erwärmt oder gekühlt werden kann, wobei zu diesem Zweck alternativ auch eine weitere Temperiervorrichtung vorgesehen sein kann.

Bevorzugt erfolgt die Methanbildung in dem Bioreaktor bei einem Druck, der höher als der Atmosphärendruck von ca. 1 bar liegt, wie er in konventionellen Anlagen zur Erzeugung von Biogas, beispielsweise Biogasanlagen oder Kläranlagen vorliegt. Besonders bevorzugt liegt der Druck in dem Bioreaktor bei 1 bar bis 30 bar, und insbesondere bevorzugt bei 2 bis 16 bar. Durch den erhöhten Druck wird die Löslichkeit der Gase Wasserstoff und Kohlendioxid bei der Gaseinbringung verbessert, was insbesondere bei Wasserstoff wichtig ist, so dass höhere Methanbildungsraten ermöglicht werden. Gemäß einer bevorzugten Ausführungsform der vorliegenden Erfindung werden nach Schritt c) und vor Schritt e) des erfindungsgemäßen Verfahrens die Schritte e01) Überführen des in Schritt c) hergestellten methan- und kohlendioxidhaltigen Biogases in einen Verdichter und e02) Verdichten des in Schritt c) hergestellten methanhaltigen Biogases in dem Verdichter auf einen Druck zwischen 1 bar und 30 bar, bevorzugt auf einen Druck zwischen 2 bar und 16 bar durchgeführt, wobei als Schritt e) der Schritt e) Überführen des in Schritt e02) verdichteten methan- und kohlendioxidhaltigen Biogases in den Bioreaktor durchgeführt wird. Das methan- und kohlendioxidhaltige Biogas wird in diesem Fall also nicht direkt in den Bioreaktor überführt, sondern zunächst in einen Verdichter geleitet, in dem dann der Druck des Gases auf den im Bioreaktor gewünschten Wert erhöht wird.

Wie bereits beschrieben dient das methan- und kohlendioxidhaltige Rohbiogas aus der Anlage zur Erzeugung von Biogas, welches einen CO₂-Anteil zwischen ca. 20 % und 55 % aufweist, als CO₂-Quelle für die biologische Methanisierung in dem Bioreaktor. Dieses Rohbiogas kann auch zunächst einer Biogasaufbereitungsanlage zugeführt werden, in der eine Trennung des methan- und kohlendioxidhaltigen Biogases in ein methanreiches Biogas und ein kohlendioxidreiches Schwachgas erfolgt. Das Schwachgas stellt üblicherweise ein Abfallprodukt der Biogasaufbereitung dar. Insbesondere wenn die Biogasaufbereitung mit den Aufbereitungsmethoden Druckwechseladsorption, Aminwäsche oder nach einem Membrantrennverfahren erfolgt, entstehen CO₂-reiche Schwachgase als Restgase, die sehr hohe CO₂-Gehalte von über 80 % bis hin zu 99,99 % haben können, also fast reinem CO₂ entsprechen. Vorteilhafterweise werden daher nach Schritt c) und vor Schritt e) des erfindungsgemäßen Verfahrens die Schritte c1) Überführen des in Schritt c) hergestellten methan- und kohlendioxidhaltigen Biogases in eine Biogasaufbereitungsanlage und c2) Trennen des in Schritt c) hergestellten methan-und kohlendioxidhaltigen Biogases in der Biogasaufbereitungsanlage in ein methanreiches Biogas und ein kohlendioxidreiches Schwachgas durchgeführt und als Schritt e) erfolgt der Schritt e) Überführen des in Schritt c2) erhaltenen kohlendioxidreichen Schwachgases in den Bioreaktor.

Wie bereits erwähnt erfolgt die Methanbildung in dem Bioreaktor bei einem Druck, der höher als der Atmosphärendruck von ca. 1 bar liegt, wie er in konventionellen Anlagen zur Erzeugung von Biogas vorliegt. Aus den bereits genannten Gründen werden daher gemäß einer bevorzugten Ausführungsform nach Schritt c2) und vor Schritt e) der oben beschriebenen bevorzugten Ausführungsform die Schritte c3) Überführen des in Schritt c2) erhaltenen kohlendioxidreichen Schwachgases in einen Verdichter und c4) Verdichten des in Schritt c2) erhaltenen kohlendioxidreichen Schwachgases in dem Verdichter auf einen Druck zwischen 1 bar und 30 bar, bevorzugt einen Druck zwischen 2 bar und 16 bar durchgeführt und als Schritt e) erfolgt der Schritt e) Überführen des in Schritt c4) verdichteten kohlendioxidreichen Schwachgases in den Bioreaktor.

Der für die biologische Methanisierung erforderliche Wasserstoff wird bevorzugt durch einen Elektrolyseur erzeugt, der in die aus einer Anlage zur Erzeugung von Biogas und einem zusätzlichen Bioreaktor bestehende Anlage integriert ist. Bevorzugt wird der Wasserstoff in dem Elektrolyseur unter Verwendung von Überschussstrom aus dem Stromnetz durch Elektrolyse aus Wasser erzeugt. Die Bereitstellung von Überschusstrom übernimmt eine Regelenergiebox, so dass die Anlage zur biologischen Methanisierung in Höhe der Leistung des entsprechenden Elektrolyseurs am Regelenergiemarkt teilnehmen kann. Auch wenn der Elektrolyseur nicht am Regelenergiemarkt teilnimmt, kann anderweitig günstiger Strom (z.B. aus speziellen Stromtarifen) verwendet werden. In diesen Fällen erfolgt die Bereitstellung von Wasserstoff nicht kontinuierlich, sondern nur in bestimmten unregelmäßigen Zeitintervallen, so dass die Wasserstoffzufuhr in den Bioreaktor zur biologischen Methanisierung diskontiniuierlich erfolgt. Alternativ kann auch Wasserstoff aus anderen Quellen wie Hydrolysegas, Synthesegas, Produktgas aus chemischen Reaktionen, H₂ biologischen Ursprungs wie z.B. durch Algen produziertes H₂ oder H₂ aus Photokatalyse zur biologischen Methanisierung eingesetzt werden.

Der Bioreaktor zur Herstellung eines methanangereicherten Gases durch biologische Methanisierung kann in Form verschiedener Reaktortypen wie Rührkesselreaktor oder Säulenreaktor ausgebildet sein. Er sollte aus druckfestem und temperaturbeständigen Material gefertigt sein, beispielsweise aus Edelstahl. Im Vergleich zu den anaeroben Fermentern einer Biogasanlage oder zu einem Faulturm einer Kläranlage ist das Reaktorvolumen des Bioreaktors zur biologischen Methanisierung deutlich kleiner, beispielsweise in einem Bereich von 1/10 bis 1/100 des Volumens eines Fermenters einer Biogasanlage oder eines Faulturms einer Kläranlage. Der Bioreaktor zur Herstellung eines methanangereicherten Gases durch biologische Methanisierung wird bevorzugt als nachrüstbare Einheit in eine bestehende Anlage zur Erzeugung von Biogas integriert und ist prinzipiell als eigenständiges Modul transportierbar. Soll nur ein Teil des vorhandenen Biogases durch biologische Methanisierung aufgewertet werden, sind auch kleinere Volumina für die separaten Bioreaktoren denkbar.

Das CO₂-haltige Biogas aus der Anlage zur Erzeugung von Biogas sowie der Wasserstoff für die biologische Methanisierung werden über Zuführleitungen in den Bioreaktor eingebracht. Vorzugsweise werden die beiden Gase bereits vor der Einbringung in den Reaktor in einer Gasmischkammer gemischt. Gemäß einer bevorzugten Ausführungsform der vorliegenden Erfindung werden daher nach Schritt e02) bzw. nach Schritt c4) und vor Schritt e) der oben beschriebenen bevorzugten Ausführungsform die Schritte c5) Überführen des in Schritt e02) bzw. in Schritt c4) verdichteten kohlendioxidreichen Schwachgases in eine Gasmischkammer, c6) Zufuhr von Wasserstoff in die Gasmischkammer und c7) Mischen bei erhöhtem Druck der in den Schritten c5) und c6) in die Gasmischkammer eingebrachten Gase durchgeführt, wobei als Schritte e) und f) der Schritt f1) Überführen der in Schritt c7) erhaltenen Gasmischung in den Bioreaktor erfolgt.

Das Volumenverhältnis der beiden, dem Bioreaktor zugeführten Eduktgase entspricht prinzipiell einem Verhältnis von H₂ zu CO₂ von 4:1. In Abhängigkeit von der Zusammensetzung des aus dem Bioreaktor entnommenen methanangereicherten Gases kann dieses Verhältnis jedoch in einem Bereich von etwa 3:1 bis 5:1, insbesondere in einem Bereich von 3,5:1 bis 4,5:1 variiert werden, wenn sich ein Restanteil bzw. Überschuss von H₂ oder CO₂ im methanangereicherten Gas nachweisen lässt. Wird im produzierten methanangereicherten Gas im Vergleich zum H₂-Anteil ein höherer Anteil nicht umgesetztes CO₂ gemessen, so wird ein stöchiometrisches Verhältnis von H₂ zu CO₂ von etwas mehr als 4:1 verwendet. Wird im Vergleich zum CO₂-Anteil ein höherer Anteil nicht umgesetzter Wasserstoff gemessen, wird ein stöchiometrisches Verhältnis von H₂ zu CO₂ von etwas weniger als 4:1 verwendet, so dass im optimalen Fall weder H₂ noch CO₂ im Produktgas neben Methan enthalten sind. In Abhängigkeit vom Vordruck der H₂ und CO₂-haltigen Eduktgase werden entsprechende Kompressoren oder Druckminderer verwendet, um die Eduktgase auf den im Bioreaktor herrschenden Druck anzupassen.

Die Zuführung der H₂- und CO₂-haltigen Eduktgase erfolgt entweder direkt über Zufuhrleitungen in den Bioreaktor, bevorzugt im unteren Bereich des Bioreaktors oder über eine Gaseinbringung in die Substratzufuhrleitung für den Bioreaktor. Vorteilhaft wird also die Zufuhr von Wasserstoff in den Bioreaktor und die Zufuhr des methan- und kohlendioxidhaltigen Biogases bzw. des kohlendioxidreichen Schwachgases in den Bioreaktor in Abhängigkeit von der Zusammensetzung des aus dem Bioreaktor entnommenen methanangereicherten Gases so geregelt, dass das molare Verhältnis von Wasserstoff zu Kohlendioxid bei der Zugabe in den Bioreaktor zwischen 3:1 und 5:1 liegt, wobei eine erhöhte Zufuhr von Wasserstoff im Falle eines erhöhten Anteils an Kohlendioxid in dem aus dem Bioreaktor entnommenen methanangereicherten Gas erfolgt und eine erhöhte Zufuhr von methan- und kohlendioxidhaltigem Biogas bzw. von kohlendioxidreichem Schwachgas im Falle eines erhöhten Anteils an Wasserstoff in dem aus dem Bioreaktor entnommenen methanangereicherten Gas erfolgt.

Als besonders geeignete Systeme zur Gaseinbringung haben sich dynamische Mischer, Mehrphasenpumpen (z.B. Edur-Pumpe) sowie Rührwerke, insbesondere Begasungsrührwerke (z.B. Ekato) und Säulenreaktoren erwiesen. Diese Gaseinbringsysteme können mit im Reaktor integrierten Systemen zur Gasfeinverteilung wie Lochplatten, Diffusoren, Sinterwerkstoffen, Membranen kombiniert werden. Damit die Gaseinbringung und -verteilung technisch gut funktioniert, sollte die Viskosität des Fermenterinhalts nicht zu hoch sein.

In unerwarteter Weise hat sich gezeigt, dass der Gärrest aus der unter mesophilen Bedingungen betriebenen Anlage zur Erzeugung von Biogas in dem unter thermophilen Bedingungen betriebenen Bioreaktor zur Methanisierung ohne große Adaptionen sowohl als Kultur- und Nährmedium für entsprechende methanogene Mikroorganismen als auch als Quelle für die geeigneten hydrogenotrophen methanogenen Mikroorganismen dienen kann. Entsprechende optionale Adaptionen des Gärrestes beschränken sich auf eine Verdünnung des Gärrestes aus der Anlage zur Erzeugung von Biogas durch Zugabe von Wasser oder wässrigen Puffer- oder Salzlösungen mit dem Ziel, im Methanisierungsmedium des Bioreaktors einen bestimmten TS-Gehalt zu erreichen. Die Anlage zur Erzeugung von Biogas liefert über das zugeführte CO₂-haltige Biogas also sowohl die CO₂-Quelle für den Bioreaktor zur biologischen Methanisierung als auch das Kultur- und Nährmedium sowie die geeigneten hydrogenotrophen methanogenen Mikroorganismen, die das gasförmig zugeführte H₂ und CO₂ zu Biomethan umsetzen.

Gemäß einer bevorzugten Ausführungsform wird dem Bioreaktor Wasser oder eine wässrige Salz- oder Pufferlösung in einer Menge zugeführt, dass der Gehalt an organischer Trockensubstanz in dem Bioreaktor von 0,5% bis 6%, bevorzugt von 1% bis 4%, besonders bevorzugt von 1,5% bis 3,5% beträgt. Besonders bevorzugt werden dem Bioreaktor zusätzlich Spurenelemente zur Anpassung der in dem Bioreaktor herrschenden Lebensbedingungen für die in dem Bioreaktor anwesende Biozönose zugeführt.

Als Substrat für den Bioreaktor zur biologischen Methanisierung eignen sich Gärreste aus jeder Art von Fermenter einer Biogasanlage und insbesondere Gärreste aus einer späteren Gärstufe oder einem Nachgärer, wobei der Gärrest aus einem Endlager, einem Gärrest- oder Substratbehälter besonders bevorzugt wird. Ebenso eignen sich als Substrat für den Bioreaktor zur biologischen Methanisierung prinzipiell jede Art von Rohschlamm und/oder Faulschlamm aus einer Kläranlage. Unter den Rohschlämmen erweist sich Überschussschlamm als besonders geeignet, obwohl er aus einer aeroben Stufe der Kläranlage stammt und die Methanisierung unter anaeroben Bedingungen stattfindet. Insbesondere bevorzugt wird Dünnschlamm aus einem Gärrestlager oder ein flüssiger Rohschlamm- und/oder Faulschlammbestandteil mit einem jeweiligen TS-Gehalt (Gehalt an Trockensubstanz gemessen in % w/w) von 0,5 % bis 7 %, bevorzugt von 1 % bis 5 %, insbesondere bevorzugt von 1,5 % bis 3,5 % eingesetzt. Da der Gehalt an organischer Trockensubstanz (oTS) in entsprechenden Gärresten oder im Rohschlamm und Faulschlamm ca. 50 bis 80 % des TS-Gehalts beträgt, ist der oTS-Gehalt entsprechend niedriger anzusetzen.

Als besonders günstig erwies sich, dass insbesondere Faulschlamm und teilweise auch Überschussschlamm bereits in der Kläranlage mit einem entsprechenden TS-Gehalt anfallen. Zusätzlich erwies sich auch der flüssige Anteil des Faulschlamms nach mechanischer Faulschlammseparation und einem TS-Gehalt von etwa 0,5 bis 1,5 % als geeignet zum Einsatz als Methanisierungsmedium. Diese mechanische Faulschlammseparation wird beispielsweise durch eine Separation mit einer Pressschnecke oder einer entsprechenden Faulschlammpresse erreicht.

Besonders bevorzugt werden daher nach Schritt c) und vor Schritt d) des erfindungsgemäßen Verfahrens die Schritte d01) Überführen zumindest eines Teils des aus der Anlage zur Erzeugung von Biogas entnommenen Gärrestes in einen Gärresteseparator und d02) Trennung des aus der Anlage zur Erzeugung von Biogas entnommenen Gärrestes in dem Gärresteseparator in einen flüssigen Gärrestbestandteil und einen festen Gärrestbestandteil durchgeführt und als Schritt d) wird der Schritt d) Überführen des in Schritt d02) erhaltenen flüssigen Gärrestbestandteils in den Bioreaktor durchgeführt.

Unter "Dünnschlamm" wird der flüssige Anteil des Gärrestes verstanden, der nach dem Abpressen des Gärrestes verbleibt, nachdem die faserhaltigen festen Anteile des Gärrestes entfernt worden sind. Dieses Abpressen wird beispielsweise durch eine Separation mit einer Pressschnecke erreicht. Da bei der Methanisierung pro Mol CH₄ jeweils zwei Mol Wasser gebildet werden, wird der Reaktorinhalt bei der biologischen Methanisierung im Laufe der Zeit verdünnt. Entsprechend wird durch eine Zufuhr von Gärrest mit entsprechendem Trockensubstanzgehalt einem zu starken Absinken des TS-Gehalts entgegengesteuert.

Im Gegensatz zu Kulturmedien aus dem Stand der Technik werden die aus der Anlage zur Erzeugung von Biogas entnommenen Gärreste, die als Methanisierungsmedium in dem Bioreaktor verwendet werden und dort gleichzeitig als Inokulum dienen, nicht autoklaviert, da sie hydrogenotrophe methanogene Mikroorganismen, die in dem Bioreaktor unter thermophilen Bedingungen die biologische Methanisierung bewirken, überraschenderweise zumindest in kleiner Anzahl bereits enthalten. Die geeigneten hydrogenotrophen methanogenen Mikroorganismen werden durch die Bedingungen in dem Bioreaktor wie hohe Temperatur, erhöhter Druck und Zufuhr von lediglich gasförmigen Edukten selektiv angereichert, während andere Mikroorganismen aus dem Gärrest abgereichert werden. Die Gärreste müssen bei entsprechender Handhabung auch nicht erst auf anaerobe Bedingungen gebracht werden, da sie teilweise bereits aus einem anaeroben Prozess stammen. Erstaunlicherweise funktioniert die biologische Methanisierung im Bioreaktor auch mit Überschussschlamm aus Kläranlagen sehr gut, obwohl dieser Klärschlamm aus aeroben Bedingungen stammt. Außerdem brauchen die Gärreste nicht synthetisch mit Hilfe einer Vielzahl von verschiedenen Salzen und Spurenelementen hergestellt werden, was insgesamt eine erhebliche Vereinfachung des Systems mit sich bringt.

Da die Gärreste aus der Anlage zur Erzeugung von Biogas als Methanisierungsmedium ausreichen, werden im laufenden Betrieb bei einem etablierten Bioreaktor weder zusätzliche Substrate noch Kulturen an methanogenen Mikroorganismen zugegeben. In bestimmten Ausnahmesituationen, wie beispielsweise der Erst-Inbetriebnahme eines Bioreaktors oder einer Wiederaufnahme des Betriebszustandes nach einer vollständigen Entleerung im Rahmen von beispielsweise Sanierungs- oder Wartungsarbeiten kann es dienlich sein, ein Inokulum mit methanogenen Mikroorganismen in Form von Kulturen oder Animpfsubstraten und/oder vorgegebene Mengen eines Substrates hinzuzugeben, um die Anlaufzeiten des Bioreaktors zu verringern. Eine derartige erstmalige bzw. einmalige Zugabe von Inokulum und/oder Substrat ist zwar in Ausnahmesituationen der genannten Art möglich, stellt jedoch keinen vorgesehenen Schritt in dem erfindungsgemäßen Verfahren dar. Schließlich ist das vorliegende Verfahren auf die Erzeugung von Methan in einem im Wesentlichen kontinuierlich betriebenen Bioreaktor gerichtet, bei dem der Bioreaktor im Betriebszustand ist und für eine stabile und zuverlässige Methanbildung allein der in den Bioreaktor zugeführte Gärrest als Methanisierungsmedium sowie die Zufuhr der gasförmigen Edukte Wasserstoff und Kohlendioxid genügt.

Der Austausch des Bioreaktorinhalts kann sowohl kontinuierlich als auch durch regelmäßigen oder unregelmäßigen batchweisen Austausch von signifikanten Anteilen des Methanisierungsmediums erfolgen. Die Austauschrate des Methanisierungsmediums in dem Bioreaktor ist im Vergleich zu Bioreaktoren mit synthetischen Kulturmedien relativ klein. Der Bioreaktor gemäß der vorliegenden Erfindung wird mit Austauschraten von weniger als 0,15/d (15 % Austausch pro Tag), bevorzugt von kleiner 0,10/d, insbesondere bevorzugt von weniger als 0,05/d oder von weniger als 0,02/d betrieben. Dabei gelten die Austauschraten für den gesamten Bioreaktorinhalt, also für das "Kulturmedium" sowie die darin enthaltenen Mikroorganismen. Gemäß einer bevorzugten Ausführungsform werden aus dem Bioreaktor pro Tag maximal 15% der Gesamtmenge des in dem Bioreaktor vorliegenden Gärrestes entnommen, besonders bevorzugt pro Tag maximal 10% der Gesamtmenge, insbesondere bevorzugt pro Tag maximal 5% der Gesamtmenge und ganz besonders bevorzugt pro Tag maximal 2%.

Eine Vorrichtung zur Rückhaltung oder Immobilisierung der methanogenen Mikroorganismen oder ein wiederkehrender oder kontinuierlicher Zusatz der methanogenen Mikroorganismen ist bei diesen Austauschraten nicht notwendig, da sich die methanogenen Mikroorganismen unter den gegebenen selektiven Bedingungen im erforderlichen Maß anreichern. Über eine Zuführvorrichtung wird das Methanisierungsmedium aus einem Vorlagebehälter, der mit Gärrest aus der Biogasanlage gespeist wird, in den Bioreaktor eingebracht. Über eine Abführvorrichtung wird ein Teil des Reaktorinhalts in einen Behälter für verbrauchtes Methanisierungsmedium abgeleitet. Da es sich bei dem Material im Wesentlichen um biogenes Restmaterial aus der angegliederten Anlage zur Erzeugung von Biogas handelt, kann es anschließend in einfacher Weise wieder in den Kreislauf der Biogasanlage, insbesondere in das Gärrestlager, zurückgeführt werden, so dass sich Synergieeffekte aus der Kombination einer Anlage zur Erzeugung von Biogas mit einer angegliederten separaten biologischen Methanisierung in dem Bioreaktor nicht nur dadurch ergeben, dass CO₂ aus dem Biogasprozess für die biologische Methanisierung verwendet wird, sondern in beiden Anlagenteilen mit demselben Biomassematerial hantiert wird. Dies vereinfacht sowohl den Umgang des Betriebspersonals mit den Biomaterialien, die Beschaffung und Entsorgung des Methanisierungsmediums sowie die Integration eines neuen Anlagenteils zur biologischen Methanisierung in eine bestehende Anlage zur Erzeugung von Biogas. Höhere Temperaturen bei der biologischen Methanisierung tragen zudem zu einer Hygienisierung des Materials bei, so dass eine Entsorgung von Methanisierungsmedium wenig problematisch ist. Vorteilhafterweise werden daher nach Schritt h) des erfindungsgemäßen Verfahrens die Schritte h1) Entnehmen des in dem Bioreaktor anfallenden Gärrestes aus dem Bioreaktor und h2) Überführen des aus dem Bioreaktor entnommenen Gärrestes in ein Gärrestlager der Biogasanlage durchgeführt.

Der Bioreaktor ist vorteilhafterweise zudem ausgestattet mit einem Druckhalteventil und entsprechend druckstabilen Komponenten, so dass ein Reaktionsdruck höher als der Atmosphärendruck, insbesondere ein Überdruck von bis zu 30 bar, bevorzugt ein Überdruck von bis zu 16 bar eingestellt werden kann.

Gemäß einer weiteren, besonders bevorzugten Ausführungsform ist der Bioreaktor mit Sensoren zur Messung von Temperatur, Druck und pH-Wert ausgestattet. Zusätzlich kann ein System zur pH-Regelung vorgesehen sein. Es hat sich gezeigt, dass in dem erfindungsgemäß betriebenen Bioreaktor insbesondere bei erhöhtem Druck und erhöhter Temperatur in einem relativ weiten pH-Bereich biologische Methanisierung stattfindet, nämlich in einem Bereich von etwa pH 5,5 bis pH 8,5. Vorteilhafterweise wird dem Bioreaktor daher Säure oder Lauge in einer Menge zugeführt, dass der pH-Wert in dem Bioreaktor von 5,5 bis 8,5, bevorzugt von 6,0 bis 7,5 beträgt.

Bei allen Gasströmen von und zu dem Bioreaktor wird sowohl die Gaszusammensetzung als auch der Gasfluss (Gasmenge pro Zeit) gemessen.

Eine Steuer- und Regelungstechnik steuert und regelt sowohl die Zu- und Abflüsse an Methanisierungsmedium sowie die Gaszu- und -abflüsse in den Bioreaktor, insbesondere auch das Verhältnis der Gasflüsse von zugegebenem Wasserstoff im Verhältnis zu zugegebenem CO₂-haltigem Gas aus der Anlage zur Erzeugung von Biogas. In der Regel wird H₂ zu CO₂ in einem Verhältnis von 4:1 eingesetzt; eine Feinregelung erfolgt jedoch nach der Zusammensetzung des Ausgangsgases aus dem Bioreaktor. Um einen möglichst hohen Biomethangehalt zu erreichen, darf weder zu viel Wasserstoff noch zu viel CO₂ im Ausgangsgas des Bioreaktors messbar sein. Entsprechend erfolgt eine Feinregelung, bei der das Verhältnis von zugegebenem H₂ zu CO₂ von dem Verhältnis von 4 : 1 nach oben oder unten etwas abweichen kann, insbesondere im Bereich von 3:1 bis 5:1, insbesondere im Bereich von 3,5:1 bis 4,5:1. Ganz besonders bevorzugt werden H₂ zu CO₂ in einem Verhältnis zwischen 4,1:1 und 3,9:1 eingesetzt

Eine Biomethanleitung führt das im Bioreaktor gebildete methanangereicherte Gas vorzugsweise am oberen Ende des Bioreaktors ab und einer weiteren Verwertung zu. In der Regel erfolgt vor einer weiteren Verwertung noch eine Gaskühlung und Gastrocknung sowie gegebenenfalls eine Entschwefelung. Entspricht die Zusammensetzung des methanangereicherten Gases nicht den Anforderungen für ein einspeisefähiges Gas, beispielsweise weil zu viel nicht umgesetztes CO₂ oder H₂ enthalten ist, wird das methanangereicherte Gas in die Biogasaufbereitungsanlage geführt. Erfüllt das gebildete methanangereicherte Gas in seiner Gaszusammensetzung Einspeisequalität, wird es direkt der Biogaseinspeiseanlage an dem jeweiligen Ort der Biomethananlage zugeführt.

Die Menge der zugeführten Eduktgase richtet sich in erster Linie nach der Menge an zur Verfügung stehendem Wasserstoff und daneben nach der Menge von CO₂, das aus der Anlage zur Erzeugung von Biogas zur Verfügung steht. Bei der Nutzung von Überschussstrom aus dem Stromnetz für die Wasserelektrolyse steht H₂ nicht kontinuierlich zur Verfügung, so dass der Bioreaktor zur biologischen Methanisierung im on/off-Betrieb genutzt wird. Dies ist bei dem erfindungsgemäßen Bioreaktor unproblematisch, da die hydrogenotrophen methanogenen Mikroorganismen in dem Methanisierungsmedium auch längere Phasen ohne Zufuhr der Eduktgase überdauern und bei einer erneuten Zufuhr von H₂ und CO₂ zeitnah mit der biologischen Methanisierung beginnen.

Neben der Einspeisung von Biomethan ins Erdgasnetz besteht die Möglichkeit einer Nutzung als Kraftstoff beispielsweise in Form einer Bioerdgastankstelle oder eine stoffliche Nutzung. Als weitere Verwertungsmöglichkeit bietet sich in Zeiten eines zu geringen Stromangebots eine Rückverstromung von Biomethan mit gleichzeitiger Wärmeerzeugung, beispielsweise über ein BHKW an. In diesem Fall benötigt man einen Zwischenspeicher für das produzierte Biomethan. Dieser wie auch ein BHKW sind jedoch häufig bereits in Biogasanlagen integriert und können so in vorteilhafter Weise für das erfindungsgemäße Verfahren genutzt werden.

In der Anlage zur biologischen Methanisierung gemäß der vorliegenden Erfindung kann in dem Bioreaktor Biomethan in einer Gasqualität von mehr als 95 % Methan, insbesondere von mehr als 97 % Methan erzeugt werden. Dies ist aufgrund der spezifischen Reaktionsbedingungen in dem Bioreaktor zur biologischen Methanisierung wie Temperatur, Druck, Mikroorganismendichte an hydrogenotrophen Methanogenen in dem Restmaterial aus der Biogasanlage sowie geeignetes Gaseinbringungssystem für H₂ und CO₂ auch mit volumenspezifischen Methanbildungsraten von mindestens von 10 Nm³/m³_{RV}d oder von mindestens 30 Nm³/m³_{RV}d oder von mindestens 50 Nm³/m³_{RV}d möglich.

Ein entscheidender Vorteil des erfindungsgemäßen Verfahrens im Vergleich zu anderen im Stand der Technik bekannten Verfahren ist, dass die Wirtschaftlichkeit erhöht wird, weil in nicht unerheblichem Maße schon vorhandene Infrastruktur und Technik sowie in einer Anlage zur Erzeugung von Biogas bereits vorhandene Biomassematerialien (Gärrest) und Gase (Rohbiogas, Schwachgas aus Biogasaufbereitung) benutzt werden, um die Methanausbeute durch einen separaten Bioreaktor zur biologischen Methanisierung zu erhöhen. Die Reaktionsbedingungen sowie die technischen Vorrichtungen zur Einbringung der Eduktgase H₂ und CO₂ unterscheiden sich in dem Bioreaktor zur biologischen Methanisierung von den Bedingungen in den konventionellen anaeroben Fermentern einer Biogasanlage oder Faultürmen einer Kläranlage, so dass hier wesentlich höhere volumenspezifische Methanbildungsraten erreicht werden können. Durch die wenig aufwändige Bereitstellung eines Methanisierungsmediums für den Bioreaktor zur biologischen Methanisierung unter Verwendung von Biomaterial aus der Anlage zur Erzeugung von Biogas werden die Kosten für die biologische Methanisierung reduziert und die Akzeptanz, eine Anlage zur Erzeugung von Biogas zu einer Anlage mit biologischer Methanisierung zu erweitern, erhöht.

### Kurze Beschreibung der Zeichnungen

Zur Illustration der Erfindung und zur Verdeutlichung ihrer Vorzüge werden nachfolgend Ausführungsbeispiele angegeben. Die Ausführungsbeispiele sollen im Zusammenhang mit den Figuren näher erläutert werden. Es versteht sich von selbst, dass die im Zusammenhang mit den Ausführungsbeispielen gemachten Angaben die Erfindung nicht beschränken sollen. Es zeigen:
- Fig. 1: Darstellung einer Ausführungsform des erfindungsgemäßen Verfahrens in Form eines Blockdiagramms;
- Fig. 2: Darstellung einer weiteren Ausführungsform des erfindungsgemäßen Verfahrens in Form eines Blockdiagramms;
- Fig. 3: in einer Diagrammdarstellung den Zeitverlauf einer biologischen Methanisierung in einem Bioreaktor über einen vorgegeben Zeitraum;
- Fig. 4A: in einer Diagrammdarstellung die Gasqualität des in einem Bioreaktor produzierten Biomethans in Abhängigkeit von der Methanisierungszeit;
- Fig. 4B: in einem Säulendiagramm den Vergleich der Gasqualität des in einem Bioreaktor produzierten Biomethans bei verschiedenen Methanbildungsraten;
- Fig. 4C: in einer Diagrammdarstellung eine zeitabhängige Biomethanisierung bei einer nicht-kontinuierlichen Zuführung von Wasserstoff.

### Wege zur Ausführung der Erfindung

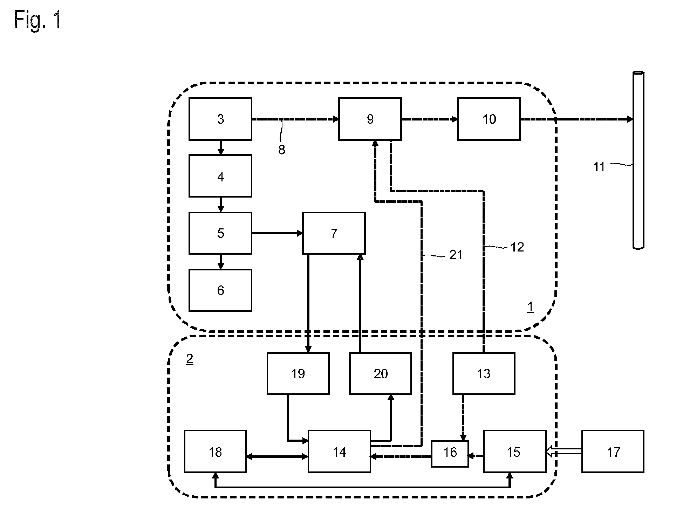

Figur 1 zeigt eine Ausführungsform des erfindungsgemäßen Verfahrens in Form eines Blockdiagramms. Mit 1 ist eine herkömmliche Biogasanlage bezeichnet. Der den Bioreaktor 14 aufweisende Anlagenteil ist mit 2 bezeichnet. Rohrleitungen, die flüssige Stoffe transportieren sind mit durchgezogenen Linien dargestellt, Rohrleitungen für Gase sind gestrichelt dargestellt.

Biogene Einsatzstoffe werden als Gärsubstrat in den anaeroben Fermenter 3 einer Biogasanlage eingebracht und dort vergoren, wobei methan- und kohlendioxidhaltiges Biogas entsteht. Die unter mesophilen Bedingungen durchgeführte Vergärung kann hierbei einstufig in nur einem Fermenter oder auch in mehreren Stufen in mehreren Fermentern erfolgen. Der Gärrest wird nach der Vergärung in ein Substratlager 4 überführt. Durch Gärvorgänge im Substratlager entstehen ebenfalls geringere Mengen an Biogas. Das vergorene Substrat aus dem Substratlager 4, welches den Gärrest darstellt, wird über eine Gärresteseparation 5 in einen festen Gärrestbestandteil und einen flüssigen Gärrestbestandteil, der auch als Dünnschlamm bezeichnet wird, aufgetrennt. Der Dünnschlamm wird in einem Dünnschlammbehälter 7 gelagert, der feste Gärrestbestandteil entsprechend in einem Behälter für festen Gärrest 6. Sowohl fester Gärrestbestandteil als auch flüssiger Gärrestbestandteil können im Anschluss zumindest teilweise dadurch weiterverwertet werden, dass sie beispielsweise von Bauern abgeholt und auf die Felder als Dünger ausgebracht werden. Das methan- und kohlendioxidhaltige Biogas aus dem Fermenter 3 sowie dem Substratlager 4 wird über eine Rohbiogasleitung 8 der Biogasaufbereitungsanlage 9 zugeführt. In der Biogasaufbereitungsanlage 9 wird zum einen ein aufbereitetes Biogas mit hohem Methananteil und als Abfallprodukt CO₂-reiches Schwachgas erzeugt. Das aufbereitete Biogas wird über eine Rohrleitung in die Biogaseinspeiseanlage 10 gebracht, wo es für eine Einspeisung in das Erdgasnetz 11 abschließend konditioniert wird.

CO₂-reiches Schwachgas aus der Biogasaufbereitungsanlage wird über die Schwachgasleitung 12 einem Verdichter 13 zugeführt, der das CO₂-reiche Schwachgas auf den entsprechenden für die biologische Methanisierung im Bioreaktor 14 notwendigen Druck bringt. Der für die biologische Methanisierung im Bioreaktor notwendige Wasserstoff wird durch einen Elektrolyseur 15 erzeugt. Da ein Großteil der gängigen Elektrolyseure Wasserstoff unter Druck zur Verfügung stellt, ist hier in der Regel kein weiterer Verdichter vor der Wasserstoffeinbringung in den Bioreaktor 14 notwendig. Je nach verwendetem Einbringsystem (nicht dargestellt) für die Wasserstoff- und CO₂-reichen Eduktgase, werden diese vor der Zufuhr in das Einbringsystem in einer Gasmischkammer 16, in der zwei Rohrleitungen zusammengeführt werden, gemischt oder als separate Gase direkt in den Bioreaktor 14 zugeführt (nicht dargestellt).

Der Elektrolyseur 15 bezieht den für die Wasserelektrolyse notwendigen Strom bevorzugt als Überschussstrom aus dem Stromnetz. Eine Regelenergiebox 17 regelt hierbei, wann günstiger Strom für die Wasserelektrolyse bezogen wird. Der Bioreaktor 14 ist mit einer Temperieranlage 18 verbunden. Einerseits muss der Bioreaktor 14 auf die für die biologische Methanisierung vorteilhafte höhere Temperatur geheizt werden, andererseits kann der Bioreaktor 14 in Zeiten der Methanisierung Wärme abgeben oder muss gekühlt werden, da die Methanbildung exotherm verläuft. In jedem Fall erfolgt die biologische Methanisierung in dem Bioreaktor 14 unter thermophilen Bedingungen. Die Abwärme des Elektrolyseurs 15 bei der Wasserelektrolyse kann ebenfalls für die Temperieranlage 18 genutzt werden. In erfindungsgemäßer Weise wird der Bioreaktor 14 mit einem Medium betrieben, das direkt aus der angrenzenden Biogasanlage entnommen wird, vorzugsweise mit dem flüssigen Gärrestbestandteil, der als Dünnschlamm bezeichnet wird. Dünnschlamm aus dem Dünnschlammbehälter 7 wird in den Vorlagebehälter 19 überführt, der als Reservoir für das Methanisierungsmedium dient, mit dem der Bioreaktor 14 für die biologische Methanisierung betrieben wird.

Um den veränderten Bedingungen in dem Bioreaktor 14 für die biologische Methanisierung gegenüber den Bedingungen in einem konventionellen Fermenter 3 einer Biogasanlage Rechnung zu tragen, kann der Dünnschlamm im Vorlagebehälter 19 adaptiert werden, so dass er als Methanisierungsmedium besser geeignet ist. Insbesondere kann der TS-Gehalt durch Zugabe eines wässrigen Mediums angepasst werden oder es kann ein hydrogenotrophe methanogene Mikroorganismen enthaltendes Inokulum hinzugefügt werden. Das Methanisierungsmedium wird aus dem Vorlagebehälter 19 in den Bioreaktor 14 zur biologischen Methanisierung über eine Rohrleitung eingebracht und nachfolgend werden bei erhöhtem Druck und bei erhöhter Temperatur die Eduktgase H₂ und CO₂ zu einem methanangereicherten Biogas umgesetzt.

Der Bioreaktor 14 ist ausgestattet mit Sensoren zur Temperatur-, Druck- und pH-Messung sowie einem System zur pH-Regelung. Der Bioreaktor 14 zur biologischen Methanisierung wird vorzugsweise kontinuierlich mit Methanisierungsmedium beschickt, jedoch mit einer im Vergleich zu synthetischen Kulturmedien kleinen Austauschrate von kleiner 0,15/d (15 % Austausch pro Tag), insbesondere von kleiner 0,10/d, insbesondere von kleiner 0,05/d, insbesondere von kleiner 0,02/d.

Über eine Abführvorrichtung wird ein Teil des Reaktorinhalts in einen Behälter für verbrauchtes Methanisierungsmedium 20 abgeleitet. Da es sich bei dem Material im Wesentlichen um biogenes Restmaterial aus der angegliederten Biogasanlage handelt, wird es anschließend wieder in den Kreislauf der Biogasanlage zurückgeführt, insbesondere in den Dünnschlammbehälter 7. Im Bioreaktor 14 gebildetes methanangereichertes Biogas wird über die Biomethanleitung 21 entweder nochmals zurück in die Biogasaufbereitungsanlage 9 geführt, wenn die Zusammensetzung des Gases nicht die Anforderungen für ein einspeisefähiges Gas erfüllt oder direkt der Biogaseinspeiseanlage 10 zugeführt (nicht dargestellt). Gegebenenfalls ist vor der Gaseinspeisung bzw. Einbringung in die Biogasaufbereitungsanlage noch eine Entwässerung und Kühlung oder Entschwefelung des erzeugten Biomethans notwendig.

Bei allen Gasströmen wird sowohl die Gaszusammensetzung als auch der Gasfluss (Gasmenge pro Zeit) gemessen. Eine Steuer- und Regelungstechnik steuert und regelt die sowohl die Zu- und Abflüsse an Methanisierungsmedium sowie die Gaszu- und -abflüsse in den Bioreaktor 14, insbesondere auch das Verhältnis der Gasflüsse von zugegebenem Wasserstoff aus dem Elektrolyseur 15 im Verhältnis zu zugegebenem CO₂-haitigem Gas aus der Biogasanlage 1. In der Regel wird H₂ zu CO₂ in einem Verhältnis von 4:1 eingesetzt; eine Feinregelung erfolgt jedoch nach der Zusammensetzung des Ausgangsgases aus dem Bioreaktor 14 in einem Verhältnis von 3:1 bis 5:1, insbesondere in einem Verhältnis von 3,5:1 bis 4,5:1. Um einen möglichst hohen Biomethangehalt zu erreichen, darf weder zu viel Wasserstoff noch zu viel CO₂ im Ausgangsgas des Bioreaktors 14 messbar sein. Die Menge der zugeführten Eduktgase richtet sich im hier beschriebenen Ausführungsbeispiel in erster Linie nach der Menge an produziertem Wasserstoff, die durch das Vorhandensein von Überschussstrom limitiert wird und durch die Regelenergiebox angezeigt wird.

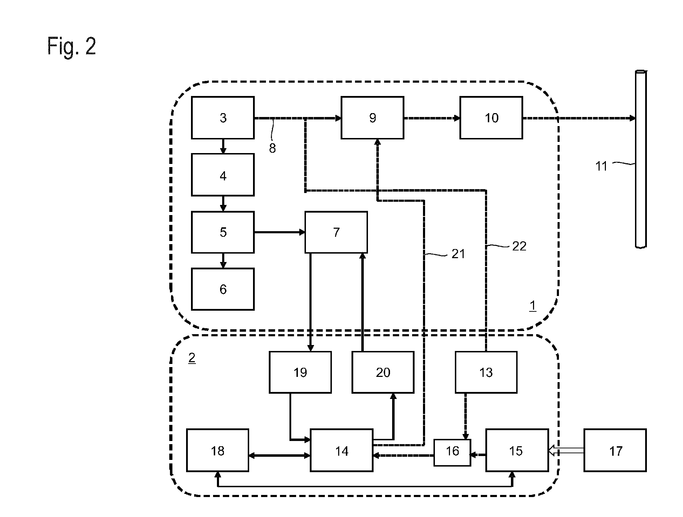

In Figur 2 ist eine weitere Ausführungsform des erfindungsgemäßen Verfahrens in Form eines Blockdiagramms dargestellt. Im Unterschied zu der in Figur 1 beschriebenen Anlage wird hier methan- und kohlendioxidhaltiges Biogas über die Rohbiogasleitung 22 dem Verdichter 13 zugeführt und zusammen mit H₂ aus dem Elektrolyseur 15 in der Gasmischkammer 16 im Verhältnis von ca. 1:4 gemischt und anschließend in den Bioreaktor 14 eingebracht. Dort wird durch die biologische Methanisierung der Methananteil des methan- und kohlendioxidhaltiges Biogases zugunsten einer Verringerung des CO₂-Anteils erhöht, so dass ein methanangereichertes Gas entsteht. Dieses wird über die Biomethanleitung 21 zurück in die Biogasaufbereitungsanlage 9 geführt, sofern die Gasqualität nicht den Einspeiseanforderungen entspricht oder direkt in die Biogaseinspeiseanlage 10 geleitet, falls die Gasqualität einspeisefähig ist (nicht dargestellt).

Alternativ oder zusätzlich zu der Biogaseinspeiseanlage 10 kann das Biogas aus der Biogasanlage und auch das methanangereicherte Gas aus dem Bioreaktor in einem BHKW verbrannt werden, wobei Strom und Wärme erzeugt werden. Der Strom aus dem BHKW wird ins Stromnetz eingespeist. Bei dieser Variante muss zusätzlich ein Gasspeicher vorhanden sein. In Zeiten von Überschussstrom darf das erzeugte Biomethan nicht über das BHKW verströmt werden, weil ohnehin genügend Strom im Netz ist. Das zusätzlich erzeugte Biomethan muss entweder zwischengespeichert oder ins Gasnetz eingespeist werden. Dafür erhöht sich durch Abschalten des Stromeinspeisers BHKW die Menge an Regelenergie, die bereitgestellt werden kann. Ist zu wenig Strom im Netz kann positive Regelenergie bereitgestellt werden, da das BHKW dann zusätzlich das zuvor erzeugte Biomethan aus dem Gaszwischenspeicher verwerten kann und so mehr Strom produziert.

In verschiedenen Beispielen werden im Folgenden experimentelle Untersuchungen und Daten zur Erzeugung von Methan mittels biologischer Methanisierung gemäß dem erfindungsgemäßen Verfahren aufgezeigt. In den folgenden Beispielen 1a bis 1d werden zunächst exemplarisch Vergleichsuntersuchungen zur Tauglichkeit von Gärresten aus Kläranlagen und Biogasanlagen als Methanisierungsmedien in separaten Bioreaktoren zur biologischen Methanisierung beschrieben. Für die vergleichenden Versuche wurden entsprechende Schlammproben in druckstabilen anaeroben Serumflaschen mit einer Mischung aus H₂ und CO₂ im Volumenverhältnis 4:1 begast und unter mesophilen sowie thermophilen Bedingungen inkubiert.

### Beispiel 1 a:

### Biologische Methanisierung mittels Faulschlamm unter mesophilen Bedingungen:

Jeweils 20 ml eines Faulschlamms bzw. Überschussschlamms aus einer unter mesophilen Bedingungen betriebenen kommunalen Kläranlage wurden mit Hilfe einer Pipette in druckstabile Serumflaschen (120 ml Volumen) gefüllt, welche mit einem Butylseptum verschlossen wurden. Anschließend wurde über eine Kanüle mit einem Schlauch an einer Gasstation drei mal Vakuum gezogen und dazwischen jeweils mit einem Eduktgasgemisch aus H₂ und CO₂ im Volumenverhältnis 4:1 begast bis ein Gasdruck von 2 bar Überdruck erreicht war. Auf diese Weise wurden anaerobe Bedingungen hergestellt und eine entsprechende Menge an den gasförmigen Edukten Wasserstoff und Kohlendioxid für die biologische Methanisierung zur Verfügung gestellt. Die Inkubation der Serumflaschen erfolgte schüttelnd bei einer mesophilen Temperatur von 37 °C.

Die Methanbildung wurde durch wiederkehrende Druckmessungen mit einem Manometer zu bestimmten Zeiten bei allen Serumflaschen beobachtet. Da sich das Gasvolumen bei der Methanisierung um einen Faktor fünf reduziert, sinkt der Druck in den Serumflaschen mit fortschreitender Methanisierung solange, bis keine Druckänderung mehr gemessen werden kann, da das Eduktgas aufgebraucht ist. Da durch wiederholtes Messen, bei dem jeweils eine Kanüle durch das Butylseptum geführt werden muss, leichte Druckverluste auch ohne Methanisierung auftreten, wurden jeweils entsprechende Kontrollproben mitgeführt, bei denen keine Druckänderung durch Methanisierung stattfinden konnte. Da es zudem eine gewisse Zeit dauert, bis sich unter den vorherrschenden Bedingungen eine aktive Mikroorganismenflora gebildet hat, die die zugegebenen Eduktgase effizient umsetzen kann, wurden jeweils mehrere Zyklen durchgeführt, bei denen nach einem vollständigen Druckabbau erneut mit H₂ und CO₂ im Volumenverhältnis 4:1 bis zu einem Überdruck von 2 bar begast wurde. Im Prinzip handelt es sich bei dieser Versuchsanordnung um Batchversuche, bei denen zwar gasförmiges Edukt wiederholt zugegeben wurde, jedoch kein weiteres Kulturmedium für die Methanogenen hinzugegeben und kein Material aus dem Versuchsansatz entnommen wurde.

Für die Versuchsansätze mit Faulschlamm wurde nicht modifizierter Klärschlamm aus dem Faulturm einer kommunalen Kläranlage verwendet. Da dieser per se einen TS-Gehalt von 4,2 %, einen oTS-Gehalt von 2,1 % sowie einen pH-Wert von 7,0 aufwies, wurde der Gärrest direkt verwendet und nicht weiter adaptiert. Der Faulschlamm wurde für die Versuche direkt eingesetzt. Zu Kontrollzwecken wurden die Serumflaschen nach der Begasung 20 Minuten bei 121 °C autoklaviert, um alle im Gärrest vorhandenen Mikroorganismen abzutöten und den so sterilisierten Faulschlamm nur als Nähr- oder Kulturmedium für extern zugesetzte Mikroorganismen zu verwenden, jedoch nicht als Inokulum. Als extern zugesetzte Mikroorganismen wurden jeweils 0,5 ml von verschiedenen Vorkulturen im Stand der Technik bekannter mesophiler Methanogener zum autoklavierten Klärschlamm hinzugegeben. Autoklavierter Faulschlamm ohne zugesetzte Mikroorganismen diente als Negativkontrolle. Die verschiedenen Versuchsansätze wurden über 7 bis 8 Zyklen inkubiert und immer wieder neu begast bis keine Erhöhung der Methanbildungsrate mehr festgestellt werden konnte. In Tabelle 1 sind die entsprechenden Versuchsergebnisse zusammengefasst.

**Tabelle 1: Methanbildung in Faulschlammproben bei 37 °C**

| Faulschlamm | Zugabe Methanogene | MBR (NI/NI_{RV}d) |
|---|---|---|
| frisch | - | ca. 8 |
| autoklaviert | - | keine Methanbildung |
| autoklaviert | Methanoculleus bourgensis | ca. 6 |
| autoklaviert | Methanobacterium espanolae | ca. 8 |
| autoklaviert | Methanobacterium formicicum | ca. 6 |
| autoklaviert | Methanobacterium bejingense | ca. 6 |

Unter den entsprechenden Versuchsbedingungen ergab sich für den direkt eingesetzten unbehandelten Faulschlamm aus einer unter mesophilen Bedingungen betriebenen Anlage ohne weitere Zugabe von methanogenen Mikroorganismen eine maximale Methanbildungsrate von etwa 8 Normliter Methan pro Normliter eingesetztem Faulschlamm pro Tag. In dem als Negativkontrolle dienenden autoklavierten Faulschlamm wurde kein Methan produziert

Erstaunlicherweise konnte durch Zusatz von Vorkulturen mesophiler Methanogener keine höhere Methanbildungsrate erreicht werden als in dem frischen Faulschlamm ohne Bakterienzugabe. Mesophiler Faulschlamm war also sowohl als Nährmedium für verschiedene hydrogenotrophe Methanogene bei der biologischen Methanisierung geeignet wie auch als Inokulum für die entsprechenden hydrogenotrophen Methanogenen, die sich unter den Bedingungen der biologischen Methanisierung einstellten.

### Beispiel 1b:

### Biologische Methanisierung mittels Überschussschlamm unter mesophilen Bedingungen:

In analoger Weise zu Beispiel 1a wurde Überschussschlamm aus einer mesophil betriebenen kommunalen Kläranlage verwendet. Im Unterschied zu Faulschlamm handelt es sich dabei um einen aeroben Schlamm, der nicht aus dem Faulturm, sondern ursprünglich aus dem belüfteten Belebtschlammbecken stammt. Im Unterschied zum Faulschlamm wurde der Überschussschlamm 1 : 2 mit Wasser verdünnt, so dass sich ein TS von 2,46 % und ein oTS von 1,54 % ergab. Der pH-Wert wurde mit Natronlauge auf pH 7,0 eingestellt. Ansonsten wurden die Versuche analog zu denen mit Faulschlamm durchgeführt. In Tabelle 2 sind die entsprechenden Versuchsergebnisse zusammengefasst.

**Tabelle 2: Methanbildung in Überschussschlamm bei 37 °C**

| Überschussschlamm | Zugabe Methanogene | MBR (NI/NI_{RV}d) |
|---|---|---|
| frisch | - | ca. 6 |
| autoklaviert | - | keine Methanbildung |
| autoklaviert | *Methanoculleus bourgensis* | ca. 5 |
| autoklaviert | *Methanobacterium espanolae* | ca. 7 |
| autoklaviert | *Methanobacterium formicicum* | ca. 6 |
| autoklaviert | *Methanobacterium bejingense* | ca. 5 |

Es erwies sich, dass sich auch Überschussschlamm aus einer unter mesophilen Bedingungen betriebenen Kläranlage als Methanisierungsmedium eignet, wobei die gemessenen Methanbildungsraten geringfügig niedriger ausfielen als bei Faulschlamm. Entsprechend zugesetzte methanogene Mikroorganismen in autoklaviertem Überschussschlamm erbrachten ebenfalls keine signifikant erhöhten Methanbildungsraten. Auch wenn die bewachsenen Kulturen von mesophilen hydrogenotrophen Methanogenen zu nicht autoklaviertem frischen Überschussschlamm zusätzlich hinzugegeben wurden, stellten sich keine höheren Methanbildungsraten ein, so dass sich unter den gegebenen Bedingungen offensichtlich bereits mit dem im Überschussschlamm vorhandenen Potential an Mikroorganismen eine optimale Population an Methanogenen für die biologische Methanisierung herausbilden konnte.

In verschiedenen Kontrollexperimenten zeigten mikroskopische Untersuchungen zudem, dass beispielsweise extern zugeführte kokkoidale oder unregelmäßige Formen von methanogenen Mikroorganismen im nicht autoklavierten Überschussschlamm im Laufe der Zeit von stäbchenförmigen methanogenen Mikroorganismen überwachsen wurden. Die extern zugesetzten methanogenen Mikroorganismen konnten sich also nicht gegen die in dem Schlamm natürlicherweise vorhandenen Mikroorganismen durchsetzen. Wurde jedoch autoklavierter Klärschlamm verwendet, vermehrten sich die extern zugesetzten methanogenen Mikroorganismen in ähnlicher Weise wie die natürlich vorkommenden methanogenen Mikroorganismen in nicht autoklaviertem Klärschlamm.

### Besipiel 1 c:

### Biologische Methanisierung mittels Faulschlamm oder Überschussschlamm unter thermophilen Bedingungen:

Die in den Beispielen 1 a und 1 b beschrieben Faulschlamm- bzw. Überschussschlammproben aus einer kommunalen Kläranlage wurden in analoger Weise zu den Beispielen 1a und 1 b in druckstabilen Serumflaschen eingesetzt und begast. Im Unterschied zu den Beispielen 1a und 1b wurden die Serumflaschen schließlich jedoch bei einer thermophilen Temperatur von 65 °C inkubiert. Im Gegensatz zu den Versuchen bei 37 °C wurden nur 4 bis 5 Zyklen durchgeführt, da im 4. Zyklus bereits die höchste Methanbildungsrate erreicht wurde. Als extern kultivierter Methanogener wurde hier ein im Stand der Technik bekannter thermophiler hydrogenotropher Mikroorganismus - *Methanothermobacter thermautotrophicus -* als Vorkultur hizugegeben, da die weiter oben erwähnten mesophilen Vertreter bei 65 °C nicht kultiviert werden konnten. Tabellen 3 und 4 fassen die entsprechenden Versuchsergebnisse bei 65 °C zusammen.

**Tabelle 3: Methanbildung in Faulschlammproben bei 65 °C**

| Faulschlamm | Zugabe Methanogene | MBR (NI/NI_{RV}d) |
|---|---|---|
| frisch | - | Ca. 18 - 20 |
| autoklaviert | - | keine Methanbildung |
| autoklaviert | *Methanothermobacter thermautotrophicus* | Ca. 13 - 20 |

**Tabelle 4: Methanbildung in Überschussschlammproben bei 65 °C**

| Überschussschlamm | Zugabe Methanogene | MBR (NI/NI_{RV}d) |
|---|---|---|
| frisch | - | Ca. 19 - 21 |
| autoklaviert | - | keine Methanbildung |
| autoklaviert | *Methanothermobacter thermautotrophicus* | Ca. 14 - 18 |

Völlig überraschend hat sich gezeigt, dass sowohl in dem an mesophile Bedingungen adaptierten Faulschlamm als auch in Überschussschlamm die biologische Methanisierung unter thermophilen Bedingungen bei 65 °C funktioniert. Unerwartet hat sich zudem erwiesen, dass unter den gegebenen Versuchsbedingungen die Methanbildungsrate um einen Faktor 2 bis 3 höher war als unter mesophilen Bedingungen, obwohl die Gärreste aus einer mesophilen Kläranlage stammten und nicht davon auszugehen war, dass eine Kläranlage für kommunale Abwässer, deren Ursprung zu einem großen Teil menschlicher Natur ist, eine gute Quelle für thermophile Mikroorganismen sein könnte.

Neben der besseren Methanbildungsrate hat sich auch gezeigt, dass die Batchkulturen unter thermophilen Bedingungen schneller das Maximum der Methanproduktion erreichten als unter mesophilen Bedingungen, nämlich bereits im vierten Begasungszyklus und nicht erst im siebten oder achten Begasungszyklus. Das bedeutet, dass sich die geeigneten Mikroorganismen unter den thermophilen Bedingungen auch relativ schnell anreicherten. Ebenso unerwartet erwies sich ein aus dem Stand der Technik als hocheffizienter thermophiler hydrogenotropher Methanbildner bekannter Mikroorganismus, nämlich *Methanothermobacter thermautotrophicus,* als keinesfalls effizienter als die Mikroorganismenflora aus den mesophilen Klärschlämmen. Die erzielten Methanbildungsraten waren mit extern zugesetztem *Methanothermobacter thermautotrophicus* eher etwas geringer als mit dem nicht autoklavierten frischen Klärschlämmen. Versuche, in denen Vorkulturen von *Methanothermobacter thermautotrophicus* in unterschiedlichen Mengen oder Konzentrationen zugegeben wurden, konnten ebenfalls nie eine höhere Methanbildungsrate aufweisen als Versuche mit frischem Faulschlamm. Lediglich in den ersten Zyklen der Methanisierung gab es bei extern zugesetztem *Methanothermobacter thermautotrophicus* etwas höhere Methanbildungsraten, da die Methanbildung etwas früher einsetzte.

### Beispiel 1 d:

### Biologische Methanisierung mittels Dünnschlamm unter mesophilen und psychrophilen Bedingungen:

Ergänzend wurde Dünnschlamm aus einer unter mesophilen Bedingungen betriebenen Biogasanlage ohne Zusatz externer Kulturen in analogen Versuchen mit druckstabilen Serumflaschen eingesetzt. Die Inkubation erfolgte hierbei bei mesophilen Temperaturen von 37 °C bzw. 25 °C und bei einer psychrophilen Temperatur von 4 °C. Während sich die erreichte maximale Methanbildungsrate unter mesophilen Bedingungen bei 25 °C und 37 °C nicht unterschied (bei 25 °C wurde lediglich ein Zyklus mehr benötigt), konnte unter psychrophilen Bedingungen bei 4 °C auch nach vielen Zyklen keine Methanbildung nachgewiesen werden.

Hier zeigte sich das erwartete Verhalten, dass ein Gärrest, der aus einer unter mesophilen Bedingungen betriebenen Biogasanlage stammt und sich dort über lange Zeit adaptiert hatte, nicht als Inokulum für eine psychrophile Anwendung der biologischen Methanisierung geeignet ist. In ähnlicher Weise hätte der Fachmann auch angenommen, dass man den mesophilen Gärrest nicht in einer thermophilen Anwendung zur biologischen Methanisierung effizient einsetzen kann, was sich in der vorliegend beschriebenen Erfindung jedoch erstaunlicherweise als geeignete vorteilhafte Maßnahme für eine Kombination von mesophiler Biogas- bzw. Kläranlage und thermophil betriebenem Bioreaktor zur biologischen Methanisierung von gasförmigen Eduktgasen erwies.

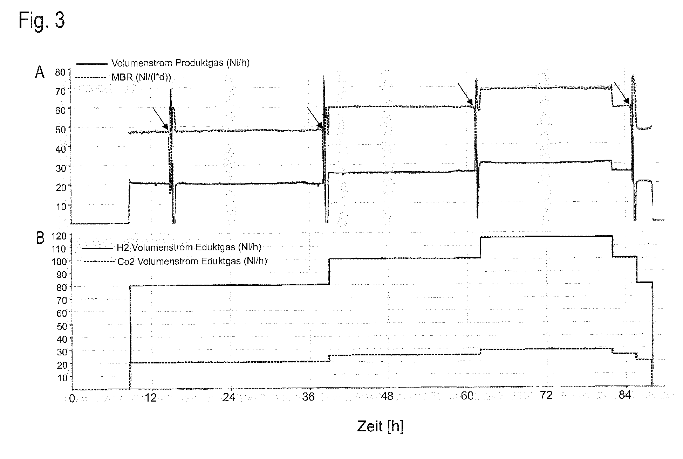

Im folgenden Beispiel 2 ist die Erzeugung von Methan mittels biologischer Methanisierung gemäß einer bevorzugten Ausführungsform des erfindungsgemäßen Verfahrens im Technikumsmaßstab aufgezeigt. Figur 3 gibt dabei den Versuchsablauf mit den zugehörigen Ergebnissen wieder.

Ein Bioreaktor (20 I Fermentervolumen) wurde mit Dünnschlamm aus einer mesophil betriebenen Biogasanlage beschickt. Der Dünnschlamm wurde zu gleichen Teilen mit Wasser verdünnt, so dass in dem Bioreaktor 11 I eines adaptierten Gärrestes mit einem TS von 3,0 % bzw. einem oTS von 2,3 als Methanisierungsmedium bereitgestellt waren. Das Methanisierungsmedium befand sich zum Versuchszeitpunkt seit 2 Wochen im Bioreaktor und war schon mehrfach mit einer Mischung aus Wasserstoff und Kohlendioxid begast worden, so dass sich eine für die biolologische Methanisierung geeignete Mikroorganismenpopulation entwickeln konnte. Es wechselten sich Zeiten, in denen mit H₂ und CO₂ im stöchiometrischen Verhältnis von 4 : 1 als Eduktgas begast wurde ab mit Zeiten, in denen der Bioreaktor keine Eduktgase erhielt. Vor dem in Figur 3 dargestellten Versuchszeitraum wurde der Reaktor zwei Tage lang nicht begast und nicht beheizt. Die Reaktortemperatur lag zu Versuchsbeginn bei 24 °C. Nach einer etwa einstündigen Aufheizphase mit einer verminderten Rührgeschwindigkeit lag die Reaktortemperatur zum Zeitpunkt 9,5 h bei 65 °C und die H₂/CO₂-Begasung wurde gestartet. Das Methanisierungsmedium wurde mit einer Rührgeschwindigkeit von 1000 Umdrehungen pro Minute gerührt und der Druck im Reaktorinneren wurde auf 1 bar Überdruck eingestellt. Der Volumenstrom der Eduktgase H₂ und CO₂ (Volumenverhältnis 4 : 1) wurde über einen Mass Flow Controller (von Bronkhorst) eingestellt. Die jeweiligen Gasvolumenströme sind im unteren Bereich der Abbildung, Fig. 3B angegeben in Normliter pro Stunde. Über den gesamten dargestellten Versuchsverlauf von 90 h wurde der Gesamteduktgasstrom (Summe aus H₂ und CO₂ Produktstrom) über den Gaseinlass in drei Stufen auf 100 NI/h bzw. 125 NI/h bzw. 145 NI/h erhöht und anschließend über kürzere Zeiträume wieder auf 125 NI/h bzw. 100 NI/h zurückgeführt, bevor die Begasung gestoppt wurde. Das Volumen des Produktgasstroms am Methangasauslass wurde mit Hilfe einer Gasuhr (von Ritter) bestimmt (dargestellt als schwarze Linie in Figur 3 unter A). Aus dem Volumen des Produktgasstroms in Verbindung mit einer Gasanalyse im Gaschromatographen und der Messung des Reaktorinhalts konnte die Methanbildungsrate in Normliter pro Liter Reaktorinhalt und Tag NI/NI_{RV}d berechnet werden (dargestellt als gestrichelte Linie in Figur 3 unter A). Zu den mit Pfeilen in Figur 3 unter A gekennzeichneten Zeiten wurde täglich jeweils 10 % des Methanisierungsmediums ausgetauscht. Da hier jeweils der Überdruck abgelassen werden musste, um frischen Gärrest einzufüllen, wurde auch die Gaszufuhr abgestellt, so dass über einen Zeitraum von knapp einer Stunde jeweils keine Produktgaswerte erhalten werden konnten. Nach dem Substratwechsel wurde zu den Zeitpunkten 39 h und 62 h der Gesamtvolumenstrom der Eduktgase von 100 NI/h auf 125 NI/h bzw. von 125 NI/h auf 145 NI/h erhöht. Zu den Zeitpunkten 82 h bzw. 86 h wurde der Gesamtvolumenstrom der Eduktgase schrittweise auf 125 NI/h bzw. 100 NI/h reduziert. Die berechneten Methanbildungsraten (MBR) lagen bei Eduktgasströmen von 100 NI/h bei etwas unter 50 NI/NI_{RV}d, bei 125 NI/h bei ca. 60 NI/NI_{RV}d, bei 145 NI/h bei knapp unter 70 NI/NI_{RV}d. Die entsprechenden Gasqualitäten lagen bei jeweils 91 % CH₄, 6 % H₂, 3 % CO₂ (MBR 50), 90 % CH₄, 7 % H₂, 3 % CO₂ (MBR 60) und 89 % CH₄, 8 % H₂, 3 % CO₂ (MBR 70).

Damit hat sich gezeigt, dass im Technikumsmaßstab über einen Zeitraum von mehreren Tagen in adaptiertem Dünnschlamm als Methanisierungsmedium ohne Zugabe externer hydrogenotropher methanogener Mikroorganismen unter den beschriebenen Versuchsbedingungen (65 °C, 1 bar Überdruck, Gaseinbringung durch Rühren) eine hohe Methanbildungsrate im Bereich von 50 NI/NI_{RV}d oder höher erreicht wird. Das dabei produzierte Biomethan weist gegenüber Rohbiogas (ca. 50 % Methan) eine gute Gasqualität im Bereich von 90 % Methan auf. Das im dargestellten Beispiel produzierte Biomethan war jedoch noch nicht einspeisefähig, da der Restgehalt an Wasserstoff mit 6 bis 8 % etwas zu hoch war.

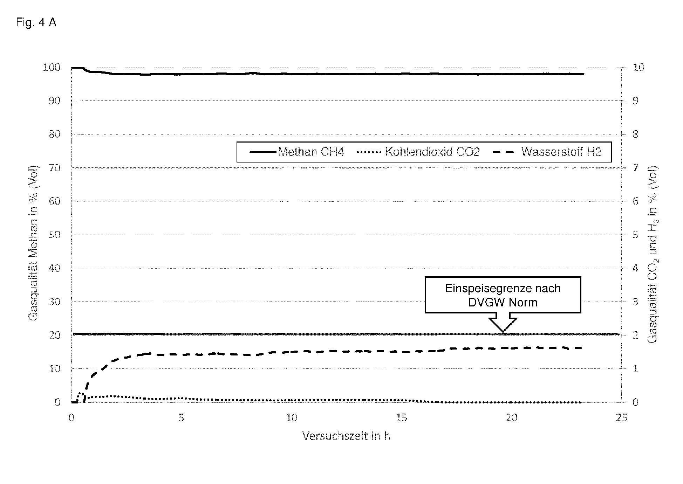

In dem weiteren Beispiel 3 ist die Erzeugung von Methan mittels biologischer Methanisierung gemäß einer weiteren bevorzugten Ausführungsform des erfindungsgemäßen Verfahrens im Technikumsmaßstab aufgezeigt. In den Figuren 4a bis 4b sind Versuchsablauf und zugehörige Ergebnisse dargestellt.

Ein Bioreaktor im Pilotmaßstab (5 m³ Reaktorvolumen) wurde als separater Reaktor zur biologischen Methanisierung an einer kommunalen Kläranlage bzw. an einer Biogasanlage installiert. Beide Anlagen zur Erzeugung von Biogas werden unter mesophilen Bedingungen betrieben. An der Biogasanlage stand sowohl Rohbiogas als auch Schwachgas aus der Biogasaufbereitung als CO₂-Quelle zur Verfügung, an der Kläranlage stand Faulgas als CO₂-Quelle zur Verfügung. Die Biogasanlage verfügt zudem über eine Gaseinspeisungsanlage, sofern das produzierte Biomethan eine entsprechende Gasqualität aufweist. Insbesondere muss der Methananteil mehr als 95 % betragen und der Wassserstoffanteil darf 2 % nicht überschreiten.

In den Figuren 4a bis 4c sind Versuche dargestellt, die am Standort der kommunalen Kläranlage durchgeführt wurden. Der Bioreaktor wurde dabei mit Faulschlamm aus einem Faulturm beschickt (Füllstand des Methanisierungsmediums im Bereich 3,1 bis 3,8 m³) und der Wasserstoff wurde durch einen PEM-Elektrolyseur zur Verfügung gestellt. Wie der Figur 4a zu entnehmen ist, kann über einen Zeitraum von über 20 Stunden unter geeigneten Reaktionsbedingungen in dem Bioreaktor durch die Methanproduktion der im Methanisierungsmedium adaptierten hydrogenotrophen Methanogenen einspeisefähiges Biomethan mit einer Methanbildungsrate von 50 Nm³/m³_{RV}d produziert werden. Die dazu eingestellten Reaktionsbedingungen waren eine Temperatur von 66 °C, ein Reaktordruck von 10,7 bar, ein TS bzw. oTS im adaptierten Klärschlamm von 3,6 % bzw. 2,1 %. Wasserstoff als Eduktgas wurde mit einem Volumenstrom von 29,0 Nm³/h zugeführt und Kohlendioxid mit einem Volumenstrom von 7,15 Nm³/h. Eine effiziente Gaseinbringung erfolgte dadurch, dass die beiden Eduktgase Wasserstoff und Kohlendioxid in der entsprechenden Stöchiometrie in einer Gaszuführleitung als Mischgas im unteren Bereich des Reaktors zugeführt und dann durch ein Begasungsrührwerk (von Ekato) mit einer Rührgeschwindigkeit von 320 Umdrehungen pro Minute in das Methanisierungsmedium eingebracht wurden. Das Verhältnis der Eduktgase lag im Bereich 4,1 : 1 bis 4,05 : 1 für Wasserstoff zu Kohlendioxid und wurde nach dem gemessenen CO₂-Restgehalt im Produktgas geregelt. Auf der linken Achse ist der Methangehalt aus der Gasanalyse dargestellt, der nach einer kurzen Anfangsphase stabil bei ca. 98 % lag. Auf der rechten Achse sind die Gasanalysewerte der Restanteile der Eduktgase Wasserstoff und Kohlendioxid dargestellt. Nach wenigen Stunden pendelten sich die Werte für H₂ bei etwa 1,5 % ein, während der CO₂-Wert auf 0 bis 0,1 % sank. Beide Werte blieben durch die Regelung über den gesamten Versuchsverlauf stabil. Insgesamt wurde über den gesamten Versuchsverlauf ein einspeisefähiges Biomethan produziert, das auch die Einspeisegrenze für Wasserstoff von 2,0 % nach DVGW-Norm erfüllte.

Eine mögliche Erklärung für die äußerst gute Gasqualität beim Start des Versuchs ist, dass der Bioreaktor einen relativ großen, mit Gas gefüllten Kopfraum besitzt. Beim Abschalten der Zufuhr der Eduktgase bleibt das Methanisierungsmedium auf diese Weise mit einem signifikanten Volumen an methanhaltigem Gas überschichtet. In dem Zeitraum, in dem keine neuen Eduktgase zugeführt werden, können die vorhandenen hydrogenotrophen Methanogenen derart weiterhin vorhandenen Restwasserstoff und vorhandenes Restkohlendioxid zu Methan umsetzen, so dass bei einem Neustart in der Regel zuerst sehr hochwertiges Biomethan aus dem Kopfraum des Reaktors abgeführt werden kann. Ein nicht zu kleiner gasgefüllter Kopfraum ist im Sinne der Erfindung vorteilhaft, da der Kopfraum als kleines Gasreservoir angesehen werden kann, das über eine gewisse Zeit nach dem Start eine gute Biomethanqualität sichert, selbst wenn in einer Anfangsphase Biomethan von weniger guter Qualität produziert werden sollte. Zudem ist ein gewisser Kopfraum insbesondere in Verbindung mit einer Gaseinbringung über ein Rührwerk technisch notwendig, da sich durch das Rühren und die damit verbundene Thrombenbildung der Füllstand apparent erhöht. In biologischen Systemen kommt es zudem öfter zu Schaumbildung, so dass bei einem hohen Füllstand im Reaktor auch die Gefahr besteht, dass Schaum in auf Produktseite nachgeschaltete Mess- und Analysegeräte eintritt und diese beschädigt oder zu einer Verfälschung der Messergebnisse führt. In vorteilhafter Weise wird hierzu ein Kopfraum über dem Methanisierungsmedium vorgeschlagen, der 10 bis 50 % des Gesamtvolumens des Reaktors ausmacht. Insgesamt muss hier eine Balance gefunden werden zwischen den höheren Investitionskosten aufgrund eines größeren Bioreaktors und den Vorteilen, die sich aus dem gasgefüllten Kopfraum ergeben.

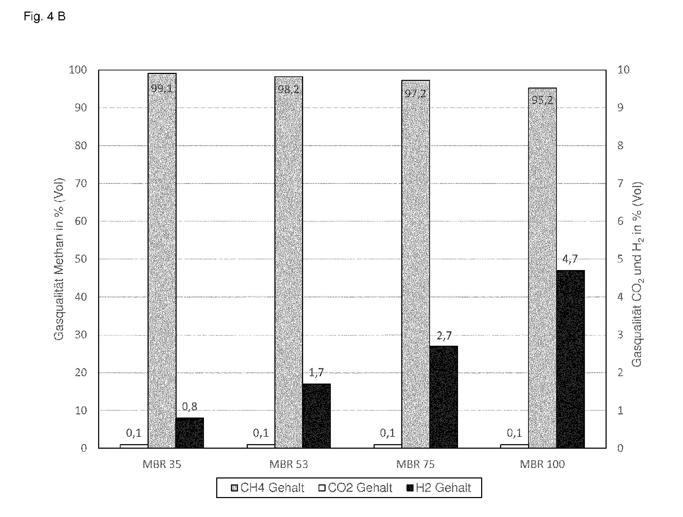

Im selben Bioreaktor wie oben beschrieben wurden bei einem Druck von 8 bar bis 12 bar, bei einer Temperatur von 65 °C, einem Füllstand von 3700 Liter und bei Stöchiometrien im Regelbetrieb für das H₂ zu CO₂-Verhältnis von 3,80 bis 4,03 durch Einbringung steigender Volumenströme an Eduktgasen steigende Methanbildungsraten (MBR) von 35 Nm³/m³_{RV}d bis 100 Nm³/m³_{RV}d erreicht. In Figur 4B ist dargestellt, wie sich die analysierten Produktgasqualitäten in Relation zu den Methanbildungsraten veränderten. Die Gasqualität für Methan ist wiederum an der linken Achse abzulesen, während die Gasqualitäten für Wasserstoff und Kohlendioxid auf der rechten Achse abgetragen sind. Bei allen Methanbildungsraten, nämlich bei 35 Nm³/m³_{RV}d (MBR 35), 53 Nm³/m³_{RV}d (MBR 53), 75 Nm³/m³_{RV}d (MBR 75) und 100 Nm³/m³_{RV}d (MBR 100) ergab sich eine hohe Gasqualität des Biomethans von über 95 % und einem sehr geringen Kohlendioxidrestgehalt von 0,1 %. Mit zunehmender Methanbildungsrate wird es jedoch zunehmend schwieriger, den Restwasserstoffgehalt des Biomethans so niedrig zu halten, dass dieses direkt einspeisefähig ist. Allerdings gibt es auch Abschnitte im Erdgasnetz, wo eine Einspeisung bis zu 5 % Wasserstoff erlaubt ist oder Verwertungsmöglichkeiten, bei denen ein geringer Restwasserstoffgehalt unproblematisch ist, wie beispielsweise die Rückverstromung des Biomethans über ein Blockheizkraftwerk. Mit der zunehmenden Bedeutung von Power-to-Gas-Anwendungen im Rahmen der Energiewende ist zudem davon auszugehen, dass sich die politischen und rechtlichen Rahmenbedingungen ändern werden.

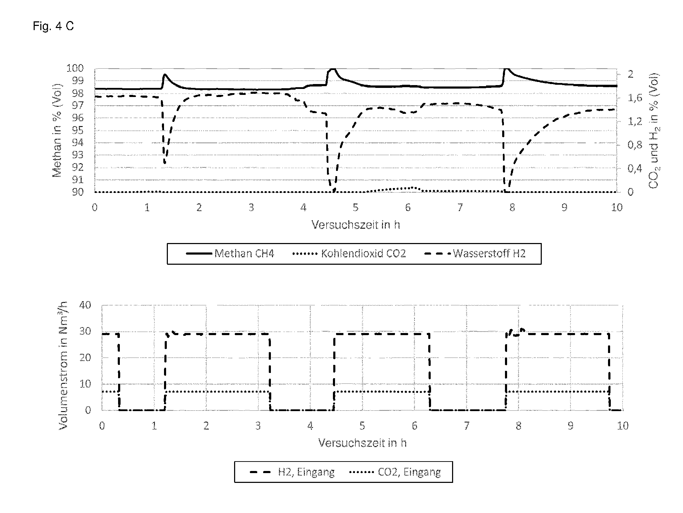

Da die biologische Methanisierung in erster Linie sinnvoll ist in Verbindung mit einer Wasserstoffproduktion durch Elektrolyse, bei der günstiger Überschussstrom eingesetzt wird, wurde in einem weiteren Experiment versucht, die Diskontinuität der Verfügbarkeit des billigen Stroms durch wiederkehrende On/Off-Versuche nachzuahmen. Die entsprechenden Versuchsergebnisse sind in Figur 4C abgebildet und wurden bei einem Druck von 10 bar, einem TS-Gehalt von 3,55 % (oTS 2,08 %), bei einer Temperatur von 65 °C und bei einer Methanbildungsrate von ca. 50 Nm³/m³_{RV}d durchgeführt. Im oberen Teil der Abbildung ist die jeweilige Gasqualität des produzierten Biomethans dargestellt, im unteren Teil der Abbildung sind die Volumenströme der Eduktgase gezeigt. In dem Versuchszeitraum von 10 Stunden wurde insgesamt 3 Mal für Zeiträume von knapp einer Stunde bis hin zu eineinhalb Stunden die Eduktgaszufuhr abgestellt. Nachdem die Eduktgaszufuhr wieder angeschaltet wurde, ergab sich über einen Zeitraum von ca. 30 Minuten bis hin zu einer Stunde die Situation, dass die Gasqualität des abgeführten Produktgases sogar noch besser war als während der laufenden Methanisierung. Dies liegt vermutlich daran begründet, dass Restwasserstoff aus dem Gasraum weiterhin abgebaut wurde und der Methangehalt des Produktgases kurzfristig auf nahezu 100 % stieg. Nach erneuter Eduktgaszugabe stellte sich wieder eine ähnliche Gasqualität ein wie vor dem Abschalten. Durch die wiederkehrenden On/Off-Versuche schien sogar eine Art Trainingseffekt aufzutreten, so dass über einen längeren Zeitraum nach der erneuten Eduktgaszufuhr eine optimale Biomethanqualität beobachtet werden konnte. Eine diskontinuierliche Begasung des Methanisierungsmediums unter den in der Erfindung angegebenen Reaktionsbedingungen erscheint unproblematisch, so dass die Nutzung von günstigem Überschussstrom oder eine Teilnahme am Regelenergiemarkt für das beschriebene Verfahren zur biologischen Methanisierung machbar erscheint.

### Bezugszeichenliste

- 1: Biogasanlage
- 2: Anlagenteil mit Bioreaktor
- 3: Fermenter
- 4: Substratlager
- 5: Gärresteseparation
- 6: Behälter für festen Gärrestbestandteil
- 7: Behälter für flüssigen Gärrestbestandteil
- 8: Rohbiogasleitung
- 9: Biogasaufbereitungsanlage
- 10: Biogaseinspeiseanlage
- 11: Erdgasnetz
- 12: Schwachgasleitung
- 13: Verdichter
- 14: Bioreaktor
- 15: Elektrolyseur
- 16: Gasmischkammer
- 17: Regelenergiebox
- 18: Temperieranlage
- 19: Vorlagebehälter
- 20: Behälter für verbrauchtes Methanisierungsmedium
- 21: Biomethanleitung
- 22: Rohbiogasleitung

## Patentansprüche

1. Verfahren zur Erzeugung von Methan umfassend die Schritte
a) Bereitstellen einer Anlage zur Erzeugung von Biogas, wobei die Anlage aufweist
- zumindest eine Vorrichtung für die Zufuhr eines Substrats,
- zumindest eine Vorrichtung für die Abfuhr eines Gärrestes,
- zumindest einen Auslass für das in der Anlage zur Erzeugung von Biogas entstehende methan- und kohlendioxidhaltige Biogas,
b) Bereitstellen eines Bioreaktors (14) zur Erzeugung eines methanangereicherten Gases, wobei der Bioreaktor (14) aufweist
- zumindest eine Vorrichtung für die Zufuhr des aus der Anlage zur Erzeugung von Biogas entnommenen Gärrestes,
- zumindest eine Vorrichtung für die Zufuhr des in der Anlage zur Erzeugung von Biogas entstehenden methan- und kohlendioxidhaltigen Biogases,
- zumindest eine Vorrichtung für die Zufuhr von Wasserstoff,
- zumindest einen Auslass für das in dem Bioreaktor entstehende methanangereicherte Gas,
- zumindest eine Temperiervorrichtung (18),
c) Herstellen von methan- und kohlendioxidhaltigem Biogas in der Anlage zur Erzeugung von Biogas, wobei das Herstellen des methan- und kohlendioxidhaltigen Biogases unter mesophilen Bedingungen erfolgt,
d) Überführen zumindest eines Teils eines aus der Anlage zur Erzeugung von Biogas entnommenen Gärrestes in den Bioreaktor (14),
e) Überführen des in Schritt c) hergestellten methan- und kohlendioxidhaltigen Biogases in den Bioreaktor (14),
f) Zufuhr von Wasserstoff in den Bioreaktor (14),
g) Herstellen von methanangereichertem Gas in dem Bioreaktor (14), wobei der in Schritt d) überführte, aus der Anlage zur Erzeugung von Biogas entnommene Gärrest als Methanisierungsmedium dient, und wobei das Herstellen des methanangereicherten Gases in dem Bioreaktor (14) unter thermophilen Bedingungen erfolgt,
h) Entnehmen des in dem Bioreaktor (14) gebildeten methanangereicherten Gases aus dem Bioreaktor.

2. Verfahren nach Anspruch 1, **dadurch gekennzeichnet, dass** es sich bei der Anlage zur Erzeugung von Biogas um eine Biogasanlage mit zumindest einem Fermenter (3) handelt, wobei der Fermenter aufweist
- zumindest eine Vorrichtung für die Zufuhr eines Substrats,
- zumindest eine Vorrichtung für die Abfuhr eines Gärrestes,
- zumindest einen Auslass für das in dem Fermenter (3) der Biogasanlage entstehende methan- und kohlendioxidhaltige Biogas.

3. Verfahren nach Anspruch 1, **dadurch gekennzeichnet, dass** es sich bei der Anlage zur Erzeugung von Biogas um eine Kläranlage mit zumindest einem Faulturm handelt, wobei der Faulturm aufweist
- zumindest eine Vorrichtung für die Zufuhr eines Rohschlamms,
- zumindest eine Vorrichtung für die Abfuhr eines Faulschlamms,
- zumindest einen Auslass für das in dem Faulturm der Kläranlage entstehende methan- und kohlendioxidhaltige Faulgas.

4. Verfahren nach einem der Ansprüche 1 bis 3, **dadurch gekennzeichnet, dass** die Temperiervorrichtung (18) derart betrieben wird, dass der in dem Bioreaktor (14) vorliegende, aus der Anlage zur Erzeugung von Biogas entnommene Gärrest auf einer Temperatur T ≥ 45 °C, bevorzugt 50 °C ≤ T ≤ 100 °C, besonders bevorzugt 60 °C ≤ T ≤ 75 °C gehalten wird.

5. Verfahren nach einem der vorhergehenden Ansprüche, **dadurch gekennzeichnet, dass** nach Schritt c) und vor Schritt e) die Schritte
e01)Überführen des in Schritt c) hergestellten methan- und kohlendioxidhaltigen Biogases in einen Verdichter (13) und
e02)Verdichten des in Schritt c) hergestellten methan- und kohlendioxidhaltigen Biogases in dem Verdichter (13) auf einen Druck zwischen 1 bar und 30 bar, bevorzugt auf einen Druck zwischen 2 bar und 16 bar durchgeführt werden und als Schritt e) der Schritt
e) Überführen des in Schritt e02) verdichteten methan- und kohlendioxidhaltigen Biogases in den Bioreaktor (14) durchgeführt wird.

6. Verfahren nach einem der Ansprüche 1 bis 4, **dadurch gekennzeichnet, dass** nach Schritt c) und vor Schritt e) die Schritte
c1) Überführen des in Schritt c) hergestellten methan- und kohlendioxidhaltigen Biogases in eine Biogasaufbereitungsanlage (9) und
c2) Trennen des in Schritt c) hergestellten methan- und kohlendioxidhaltigen Biogases in der Biogasaufbereitungsanlage (9) in ein methanreiches Biogas und ein kohlendioxidreiches Schwachgas durchgeführt werden und als Schritt e) der Schritt
e) Überführen des in Schritt c2) erhaltenen kohlendioxidreichen Schwachgases in den Bioreaktor (14)
durchgeführt wird.

7. Verfahren nach Anspruch 6, **dadurch gekennzeichnet, dass** nach Schritt c2) und vor Schritt e) die Schritte
c3) Überführen des in Schritt c2) erhaltenen kohlendioxidreichen Schwachgases in einen Verdichter (9) und
c4) Verdichten des in Schritt c2) erhaltenen kohlendioxidreichen Schwachgases in dem Verdichter (9) auf einen Druck zwischen 1 bar und 30 bar, bevorzugt einen Druck zwischen 2 bar und 16 bar
durchgeführt werden und als Schritt e) der Schritt
e) Überführen des in Schritt c4) verdichteten kohlendioxidreichen
Schwachgases in den Bioreaktor (14)
durchgeführt wird.

8. Verfahren nach einem der Ansprüche 5 oder 7, **dadurch gekennzeichnet, dass** nach Schritt e02) oder nach Schritt c4) und vor Schritt e) die Schritte
c5) Überführen des in Schritt e02) verdichteten methan- und kohlendioxidhaltigen Biogases oder in Schritt c4) verdichteten kohlendioxidreichen Schwachgases in eine Gasmischkammer (16),
c6) Zufuhr von Wasserstoff mit entsprechenden Druck in die Gasmischkammer (16) und
c7) Mischen bei erhöhtem Druck der in den Schritten c5) und c6) in die Gasmischkammer (16) eingebrachten Gase
durchgeführt werden und als Schritte e) und f) der Schritt
f1) Überführen der in Schritt c7) erhaltenen Gasmischung in den Bioreaktor (14)
durchgeführt wird.

9. Verfahren nach einem der Ansprüche 1 bis 8, **dadurch gekennzeichnet, dass** nach Schritt c) und vor Schritt d) die Schritte
d01)Überführen zumindest eines Teils des aus der Anlage zur Erzeugung von Biogas entnommenen Gärrestes in einen Gärresteseparator (5) und
d02)Trennung des aus der Anlage zur Erzeugung von Biogas entnommenen Gärrestes in dem Gärresteseparator (5) in einen flüssigen Gärrestbestandteil und einen festen Gärrestbestandteil
durchgeführt werden und als Schritt d) der Schritt
d) Überführen des in Schritt d02) erhaltenen flüssigen Gärrestbestandteils in den Bioreaktor (14)
durchgeführt wird.

10. Verfahren nach einem der Ansprüche 1 bis 9, **dadurch gekennzeichnet, dass** das in Schritt h) aus dem Bioreaktor (14) entnommene methanangereicherte Gas in Bezug auf die enthaltenen Anteile an Methan, Wasserstoff und Kohlendioxid analysiert wird und die Zufuhr von Wasserstoff in den Bioreaktor (14) und die Zufuhr des methan- und kohlendioxidhaltigen Biogases oder des kohlendioxidreichen Schwachgases in den Bioreaktor (14) in Abhängigkeit von der Zusammensetzung des aus dem Bioreaktor (14) entnommenen methanangereicherten Gases so geregelt erfolgt, dass das molare Verhältnis von Wasserstoff zu Kohlendioxid bei der Zugabe in den Bioreaktor (14) zwischen 3:1 und 5:1 liegt, wobei eine erhöhte Zufuhr von Wasserstoff im Falle eines erhöhten Anteils an Kohlendioxid in dem aus dem Bioreaktor (14) entnommenen methanangereicherten Gas erfolgt und eine erhöhte Zufuhr von methan- und kohlendioxidhaltigem Biogases bzw. von kohlendioxidreichem Schwachgas im Falle eines erhöhten Anteils an Wasserstoff in dem aus dem Bioreaktor (14) entnommenen methanangereicherten Gas erfolgt.

11. Verfahren nach einem der Ansprüche 1 bis 10, **dadurch gekennzeichnet, dass** dem Bioreaktor (14) Wasser oder eine wässrige Salz- oder Pufferlösung in einer Menge zugeführt wird, dass der Gehalt an organischer Trockensubstanz in dem Bioreaktor (14) von 0,5% bis 6%, bevorzugt von 1% bis 4%, besonders bevorzugt von 1,5% bis 3,5% beträgt.

12. Verfahren nach einem der Ansprüche 1 bis 11, **dadurch gekennzeichnet, dass** dem Bioreaktor (14) Spurenelemente zur Anpassung der in dem Bioreaktor herrschenden Lebensbedingungen für die in dem Bioreaktor anwesende Biozönose zugeführt werden.

13. Verfahren nach einem der Ansprüche 1 bis 12, **dadurch gekennzeichnet, dass** nach Schritt h) die Schritte
h1) Entnehmen des in dem Bioreaktor (14) anfallenden Gärrestes aus dem Bioreaktor (14) und
h2) Überführen des aus dem Bioreaktor (14) entnommenen Gärrestes in ein Gärrestlager der Anlage zur Erzeugung von Biogas
durchgeführt werden.

14. Verfahren nach einem der Ansprüche 1 bis 13, **dadurch gekennzeichnet, dass** das Überführen des in der Anlage zur Erzeugung von Biogas hergestellten Biogases in den Bioreaktor in Schritt e) sowie das Zuführen von Wasserstoff in den Bioreaktor in Schritt f) oder das Überführen der in Schritt c7) erhaltenen Gasmischung in den Bioreaktor in Schritt f1) mittels eines Begasungsrührwerkes erfolgt.

15. Verfahren nach einem der Ansprüche 1 bis 14, **dadurch gekennzeichnet, dass** in Schritt b) ein Bioreaktor mit einem derart ausgelegten Fassungsvolumen bereitgestellt wird, dass 10% bis 50% des Fassungsvolumens des Bioreaktors als ein mit Gas füllbarer Kopfraum oberhalb des in dem Bioreaktor vorliegenden Gärrestes zur Verfügung gestellt wird.
